# EUROPEAN PATENT SPECIFICATION

(11) **EP 4 321 186 B1**
(45) Date of publication and mention of the grant of the patent: **12.06.2024**
(21) Application number: 23195489.2
(22) Date of filing: 25.03.2022
(51) Int. Cl.: A61M 1/36

(54) **DEVICES FOR FISTULA-FREE HEMODIALYSIS**
VORRICHTUNGEN ZUR FISTELFREIEN HÄMODIALYSE
DISPOSITIFS D'HÉMODIALYSE SANS FISTULE

(30) Priority: 30.03.2021 US 202163167771 P; 31.03.2021 US 202163168276 P; 31.03.2021 US 202163168277 P; 15.07.2021 US 202163222062 P
(43) Date of publication of application: 14.02.2024
(62) Divisional of application: 22720927.7
(73) Proprietor: GRUNWALD, Sorin, 93500 Pantin (FR)
(72) Inventor: GRUNWALD, Sorin, 93500 Pantin (FR)

(56) References cited:
- US-A1- 2005 143 758
- US-A1- 2014 100 510
- US-A1- 2020 114 060

## Description

### REFERENCES CITED

### CROSS REFERENCE TO RELATED APPLICATIONS

This application is a division of PCT Application No. PCT/EP2022/057987 filed on 25 March 2022 now given the European Application No. 22720927.7, International Publication Number WO 2022/207505 A2 and claims priorities to:
1. U.S. Provisional Patent Application Serial No. US 63/167,771 filed on March 30, 2021 (Needleless transcutaneous port)
2. U.S. Provisional Patent Application Serial No. US63/168,276, filed on March 31, 2021 (Anastomosis device and method for percutaneous delivery thereof)
3. U.S. Provisional Patent Application Serial No. US63/168,277, filed on March 31, 2021 (Devices and methods for Hemodialysis)
4. U.S. Provisional Patent Application Serial No. US 63/222,062, filed on July 15, 2021 (Devices and methods for sutureless anastomosis).

### U.S. PATENT DOCUMENTS

| | | | |
|---|---|---|---|
| 3,628,813 | 12/1971 | Lee | Fluid connecting device |
| 4,007,743 | 2/1977 | Blake | Opening mechanism for umbrella-like intravascular shunt defect closure device |
| 4,352,358 | 10/1982 | Angelchik | Apparatus for effecting anastomotic procedures |
| 4,353,368 | 10/1982 | Slovak et al. | Device for hemodialysis |
| 4,569,675 | 2/1986 | Prosl et al. | Transcutaneous infusion system |
| 4,892,518 | 1/1990 | Cupp et al. | Hemodialysis |
| 4,968,422 | 11/1990 | Runge et al. | Pulsatile flow hemodialysis |
| 5,035,702 | 7/1991 | Taheri | Method and apparatus for providing an anastomosis |
| 5,342,393 | 8/1994 | Stack | Method and device for vascular repair |
| 5,456,712 | 10/1995 | Maginot | Graft and stent assembly |
| 5,562,617 | 10/1996 | Finch, Jr. et al. | Implantable vascular device |
| 5,755,775 | 5/1998 | Trerotola et al. | Percutaneous stent-graft and method for delivery thereof |
| 5,755,778 | 5/1998 | Kleshinski | Anastomosis device |
| 5,861,003 | 1/1999 | Latson et al. | Apparatus and method for occluding a defect or aperture within body surface |
| 5,911,706 | 6/1999 | Estabrook et al. | Device for subcutaneous accessibility |
| 5,944,688 | 8/1999 | Lois | Implantable hemodialysis access port assembly |
| 6,036,702 | 3/2000 | Bachinski et al. | Medical grafting connectors and fasteners |
| 6,231,541 | 5/2001 | Kawamura | No-needle blood access device for hemodialysis and no-needle connecting cannula assembly |
| 6,312,446 | 11/2001 | Huebsch et al. | Apparatus and method for closing a septal defect |
| 6,402,764 | 6/2002 | Henricksen et al. | Everter and threadthrough system for attaching graft vessel to anastomosis device |
| 6,428,550 | 8/2002 | Vargas et al. | Sutureless closure and deployment system for connecting blood vessels |
| 6,648,901 | 11/2003 | Fleischman et al. | Anastomosis system |
| 6,719,781 | 4/2004 | Kim | Catheter apparatus having an improved shape-memory alloy cuff and inflatable on-demand balloon for creating a bypass graft in-vivo |
| 6,776,785 | 8/2004 | Yencho et al. | Implantable superelastic anastomosis device |
| 7,585,306 | 9/2009 | Abbott et al. | Anastomosis device, tools and methods of using |
| 7,762,977 | 7/2010 | Porter et al. | Device and methods for vascular access |
| 8,277,465 | 10/2012 | Beane et al. | Apparatus and method for connecting a conduit to a hollow vessel |
| 8,747,344 | 6/2014 | Khan | Hybrid arteriovenous shunt |
| 8,845,573 B2 | 9/2014 | Häusler et al. | Implantable access for removal and/or return of fluids; reference cited in the written opinion of the International Searching Authority [19] |
| 8,900,288 | 12/2014 | Chobotov et al. | Advanced endovascular graft delivery system and method of treatment |
| 8,926,591 | 1/2015 | Schutz et al. | Implantable vascular access |
| 9,247,930 | 2/2016 | Coleman et al. | Device and methods for occluding or promoting fluid flow |
| 9,597,443 | 3/2017 | Yevzlin et al. | Anastomotic connector |
| 9,820,743 | 11/2017 | Asfora et al. | Sutureless vascular anastomosis connection |
| 10,702,688 | 7/2020 | Mason et al. | Vascular access device |
| 10,166,321 | 1/2019 | Casiello et al. | High-flow port and infusion needle systems |
| 10,751,056 | 8/2020 | Porter | Methods and apparatus for percutaneous bypass graft |
| 10,773,010 | 9/2020 | Phillips et al. | Subcutaneous vascular access ports and related systems and methods (needle guiding devices) |
| 10,835,366 | 11/2020 | Donadio, III et al. | Arterial and venous anchor devices forming an anastomotic connector and system for delivery |
| 10,835,663 | 11/2020 | Peh et al. | Subcutaneous implantable device for guiding a vascular access member and method for vascular access |
| US 2005/143758 A1 (ABBOT RYAN [US] ET AL) | | | 30 June 2005, reference cited in the written opinion of the International Searching Authority [19]. |
| US 2014/100510 A1 (YEVZLIN ALEXANDER S [US] ET AL) | | | 10 April 2014, reference cited in the written opinion of the International Searching Authority [19] |
| US 2020/114060 A1 (VARTANIAN SHANT [US] | | | 16 April 2020, reference cited in the written opinion of the International Searching Authority [19] |

### FOREIGN PATENT DOCUMENTS

| | | | | |
|---|---|---|---|---|
| WO | 97/42878 | 11/1997 | Kotula et al. | Percutaneous catheter directed intravascular occlusion devices |
| WO | 2020/004672 A1 (KK ADVANCE [JP]) | | | 2 January 2020 (2020-01-02) reference cited in the written opinion of the International Searching Authority [19] |

### OTHER PUBLICATIONS

1. Agarwal A. K. et al., Innovations in vascular access for hemodialysis, Kidney International (2019) 95, 1053-1063
2. Allon M., Vascular Access for Hemodialysis Patients - New Data Should Guide Decision Making, American Society of Nephrology, CJASN Vol 14 June, 2019
3. Canaud B. et al., Vascular Access Management for Haemodialysis: A Value-Based Approach from NephroCare Experience, IntechOpen 2019
4. Ilie V. et al., Sutureless Microvascular Anastomosis: Literature Review, International Microsurgery Journal, 2019; 3(2):1
5. Mallios A. et al., Early results of percutaneous arteriovenous fistula creation with the Ellipsys Vascular Access System, Journal of Vascular Surgery, 2018
6. Al-Jaishi A.A. et al., Complications of the Arteriovenous Fistula: A Systematic Review, J Am Soc Nephrol 28: 1839-1850, 2017
7. Al-Balas A. et al., The Clinical and Economic Effect of Vascular Access Selection in Patients Initiating Hemodialysis with a Catheter, J Am Soc Nephrol 28: 3679-3687, 2017
8. Al Shakarchi J. et al., Early cannulation grafts for hemodialysis: a systematic review, J Vasc Access 2015; 16(6): 493-497
9. Parisotto, M.T., et al., Cannulation technique influences arteriovenous fistula and graft survival, Kidney International (2014) 86, 790-797
10. Ebner A. et al., Minimally Invasive Sutureless Anastomosis of an AV Hemodialysis Graft, Endovascular Today, November 2014
11. Stolic R, Most Important Chronic Complications of Arteriovenous Fistulas for Hemodialysis, Medical Principles and Practice 2013; 22:220-228
12. Vachharajani T.J., Atlas of Dialysis Vascular Access, Wake Forest University, School of Medicine, 2010
13. Hadaway L. et al., Needleless Connectors: A Primer on Terminology, Journal of Infusion Nursing, January 2010
14. Misra M., The basics of hemodialysis equipment, Hemodialysis International 2005; 9:3--36
15. Twardowski Z.J. et al., Blood Flow, Negative Pressure, and Hemolysis During Hemodialysis, Home Hemodial Int, Vol 3, 45-50. 1999
16. Foran R.F., Quinton-Scribner Cannulas for Hemodialysis - Review of Four Years' Experience, The Western Journal of Medicine, March 1970, 112-3
17. Brescia M.J. et al., Chronic hemodialysis using venipuncture and a surgically created arteriovenous fistula, N Engl J Med. 1966; 275:1089-1092
18. Clark P.B., Routine Use of Scribner Shunt for Haemodialysis, British Medical Journal, May 1966: 1200 - 1202
19. PCT, INTERNATIONAL SEARCH REPORT AND WRITTEN OPINION OF THE INTERNATIONAL SEARCHING AUTHORITHY mailed on 17 November, 2022 for International Application No. PCT/EP2022/057987, International Publication Number WO 2022/207505 A2.

### TECHNICAL FIELD

The present invention relates generally to devices and methods that can be used for vascular access for hemodialysis. More specifically, the present invention relates to devices and methods that can be delivered percutaneously and suturelessly and that do not require an arteriovenous fistula, an arteriovenous graft, or a central venous catheter for vascular access (fistula-free hemodialysis). Further, the present invention relates to devices for improved and needleless connection of a patient to a dialyzer.

### BACKGROUND

### Clinical Background

Worldwide, hemodialysis (HD) remains the prevalent dialysis modality for more than 2 million patients with end-stage renal disease. The health care expenditure to treat end-stage renal disease has increased to approximately $34 billion in 2015 in the United States alone. A significant portion of this expense is related to the establishment and maintenance of vascular access (VA) for HD [1].

The 2006 Kidney Disease Outcomes Quality Initiative (KDOQI) VA guidelines state that "options for fistula placement should be considered first." However, more recent publications have reported 30%-60% arteriovenous fistula (AVF) nonmaturation rates for AVFs that cannot be used or require assisted maturation [2]. Although the AVF is currently considered the ideal model of VA for HD, AVFs are the source of complications, the most important being lymphedema, infection, aneurysm, stenosis, congestive heart failure, steal syndrome, hand ischemia and thrombosis. Fistula complications are associated with morbidity, mortality, and a high economic burden [6]. Several studies indicate that about 30% of HD hospitalizations are caused by VA construction and complications of vascular access [11]. Endovascular creation has simplified the creation of AVFs using either radiofrequency (everlinQ, TVA Medical, Austin, TX, USA) or thermal resistance (Ellipsys, Avenu Medical, San Juan Capistrano, CA, USA) arteriovenous anastomosis devices [1], [5]. However, many of clinical issues and complications related to AVFs, as mentioned above, have remained unaddressed.

If a patient is not a suitable candidate for AVF, arteriovenous grafts (AVG) is the second vascular access option for HD. Compared to the AVF, the AVG has better mechanical strength, earlier use, and primary failure rates but also increased risk of developing graft stenosis, a fivefold increase in infection risk, a poorer long-term patency, higher levels of complications and requires more interventions than AVF [2]. Central venous catheters (CVC) are also used in specific cases for starting hemodialysis because they provide immediate vascular access. However, prolonged CVC use results in high risk of CVC-related bloodstream infections. CVCs are also associated with a higher risk of central vein stenosis and decreased survival rates [2].

### Related Art

The creation of an AVF for long-term HD in 1966 by Cimino and Brescia [17] was the first major innovation after the Scribner shunt first described in 1961 and was followed by the development of arteriovenous grafts and hemodialysis catheters. To date, the innovations in vascular access for hemodialysis have focused on arteriovenous fistulas, arteriovenous grafts, central venous catheters and the process of healthcare. Innovations related to arteriovenous fistulas have focused on: a) endovascular creation; b) (sutureless) anastomotic devices; c) maturation facilitators; and d) superificialization. Innovations related to arteriovenous grafts have focused on: a) early access AVG; b) heparin-bonded AVG; c) hybrid catheter graft; d) hybrid graft-stent; and e) tissue bioengineered vessels. Innovations related to central venous catheters have focused on: a) catheter tip design; b) catheter coating; and c) catheter lock solutions. Innovations related to the process of healthcare have focused on: a) patient-centric care; b) value versus volume-based care; c) preemptive versus reactive care; and d) evidence-based care to reduce health care-associated infections [1].

Foran [16] and Clark [18] describe the use of a device, the Scribner shunt, that provides separate arterial and venous access for HD. Some of the most important benefits of the Scribner shunt are: a) the ease-of-use for repetitive HD access compared to new cannulations; and b) the ability to use the shunt for HD access immediately after implantation. Some of the limitations of the Scribner shunt were: a) the fact that the device was an extracorporeal device which lead to a higher risk of infection, in particular at the points of insertion; b) the arterial and venous indwelling segments of the devices caused thrombosis and stenosis; c) the high complexity of the surgical insertion and fixation of the cannula tip, particularly in the artery; and d) the material of the device caused clotting and progressive deterioration of the cannula function.

In U.S. Pat. No. 6,231,541 Kawamura describes a no-needle blood access device for HD comprising an artificial conduit whose opposite ends are anastomosed to a target artery or vein. The device contains a transcutaneous component that allow for connecting the subcutaneous conduit to an extracorporeal HD machine. Some of the limitations of Kawamura's disclosure are: the disclosed device requires a fistula or graft to connect a vein to an artery, the fistula/graft is created through open surgery and sutured to the target vessels; there is only one skin access point for both the arterial and venous lines for hemodialysis limiting the choice of target blood vessels and access points; the use of disclosed shutters to open and close conduits leads to blood loss when connecting a dialyzer; the long cannulas inserted in the blood stream increase the risk of infections; and once implanted, the device cannot be replaced if it fails in time.

In U.S. Pat. No. 6,719,781 Kim describes a catheter apparatus, an improved introducer system, and a methodology for creating a bypass using a prepared shape-memory alloy cuff and a graft segment in tandem as a shunt. Some of the limitations of Kim's disclosure are: the cuff is fixed to the graft by the surgeon through suturing during the procedure and the fixing means can cause tissue damage; the cuff expands based on thermal properties and needs cooling until deployment; the disclosed device can only be used for large arteries (aorta) at one end of the graft while the other end of the graft is connected surgically with sutures by the surgeon in a standard surgical procedure; and, in general, grafts cannot be connected to a dialyzer considering the conduit sizes and fluid pressures required by the hemodialysis process.

In U.S. Pat. No. 6,776,785 Yencho et al. describe a one-piece anastomosis device formed of a superelastic or pseudoelastic material which self-deforms or self-deploys from an insertion configuration to a tissue holding configuration. The self-deploying anastomosis device does not rely on a temperature transformation to achieve deployment. Some of the limitations of Yencho's disclosure are: the disclosed device can only be used to connect a tissue graft and cannot be used to connect a catheter or other semi-rigid conduit and, thus, cannot be used for hemodialysis; the risk of damaging the blood vessel is high during the deployment procedure due to the lack of maneuverability, and the device cannot be placed without direct visualization of the target vessel.

In U.S. Pat. No. 7,585,306 Abbott et al. describe anastomosis devices, tools and methods of performing sutureless anastomosis. Anastomosis devices are provided for fixing a first conduit to a second conduit in an anastomosis, where the conduits are joined by interfacing their inner walls together. Some of the limitations of Abbott's disclosure are: the devices can be used only for large blood vessels, e.g. aorta, because of the reverted graft attaching mechanism; the device cannot be connected to a catheter or other semi-rigid conduit and, thus, cannot be used for hemodialysis;

In U.S. Pat. Nos. 2014/100510 and 9,247,930 Coleman et al. describe devices and sutureless percutaneous methods for occluding or promoting fluid flow through openings. In an exemplary embodiment an occlusion device is provided having an outer elongated tubular body that is configured to expand and form proximal and distal wings proximate to opposed ends of the opening. The device can include a component to occlude flow through the tubular body and thus through the opening. Some of the limitations of Coleman's disclosure are: the disclosed device cannot be used to promote flow in small vessels because of the inherent wall thickness of the disclosed device structure. A device with a small outer diameter and an even smaller inner diameter cannot ensure the fluid debits required for hemodialysis, i.e., 300 - 600 ml/min; in addition, when promoting flow, certain disclosed device components must reside inside the target blood vessel obstructing flow in said blood vessel. Thus, the device can only be used for bypass procedure where the blood flow is already obstructed in the target vessel. Moreover, the disclosed device is made of a solid stainless steel structure that is too heavy to attach to small vein walls that would collapse under the device weight.

In U.S. Pat. No. 9,597,443 Yevzlin et al. describe an anastomotic connector comprising a tubular access port and an anchor with a plurality of fingers to be extended in a blood vessel connected to the tubular access port. Some of the limitations of Yevzlin's disclosure are: the disclosure does not allow for percutaneous deployment of a catheter pre-connected to the disclosed anastomotic connector thus limiting the choice of anastomosis locations to those directly accessible by open surgery; the proximal end of the anastomotic connector that resides outside the blood vessel is fixed in shape and perpendicular to the longitudinal axis of the blood vessel, said characteristics further limiting the maneuverability of the device and the ability to connect it to smaller blood vessels.

### Clinical Need

What is needed are improved devices and methods for vascular access for hemodialysis that reduce the risks associated with establishing and maintaining vascular access, decrease time to first use, increase long-term patency, minimize complications, simplify the hemodialysis procedure, and reduce the burden on patients and on the healthcare system.

### SUMMARY

Devices and methods for fistula-free vascular access for hemodialysis are disclosed herein. In exemplary embodiments, devices are provided including a transcutaneous access port for needlelessly connecting a patient to a dialyzer, an intracorporeal conduit providing a fluid path between said transcutaneous port and a blood vessel, and a device anchor, said device anchor permitting the sutureless anastomosis of said intracorporeal conduit to said blood vessel. In exemplary embodiments, methods are provided for creating fistula-free vascular access for hemodialysis and performing hemodialysis procedures without needle punctures.

In one exemplary embodiment, an implantable transcutaneous access port disclosed herein comprises a port body with an inner lumen and a subcutaneous segment attachable to an intracorporeal conduit, wherein the longitudinal axis of said subcutaneous segment and the longitudinal axis of said port body are at a variable angle with respect to each other in order to adapt to the anatomy of the access and implant locations and wherein said inner lumen has different profiles such as to provide variable blood flow characteristics adapted to either arterial or venous blood flow and, wherein the port body can be connected to an extracorporeal connector and to a dialyzer.

In one exemplary embodiment, an intracorporeal conduit disclosed herein comprises a distal more rigid segment and a proximal less rigid segment, said proximal less rigid segment being attached to a transcutaneous access port and said distal more rigid segment being fixedly attached to and within a tubular device anchor, wherein said distal more rigid segment extends through the inner lumen of said device anchor such that the distal end of said segment and the distal end of said device anchor are flush, and wherein the outer diameter of said distal more rigid segment of said conduit is equal to or slightly smaller than the inner diameter of said device anchor.

In one exemplary embodiment, a device anchor disclosed herein for suturelessly anastomosing the distal end of an intracorporeal conduit to a blood vessel or graft comprises a tubular body with a pre-shaped self-expandable proximal segment, a pre-shaped self-expandable waist segment, and a pre-shaped self-expandable distal segment, wherein said proximal segment self- expands outside a wall of said blood vessel or graft, the length of the said waist segment is approximately the size of the thickness of said wall and said distal segment self-expands inside said blood vessel or graft or inside a tubular device implanted in the blood vessel or graft as to, together with the self-expanded proximal segment, apply sufficient pressure on the vessel wall in order to keep said device steadily attached to said wall without penetrating it, wherein the proximal end of said device anchor slides freely over said distal more rigid segment of said intracorporeal conduit during compression of said device anchor and during self-expansion of said device anchor from a compressed configuration to its pre-shaped expanded configuration, and wherein said device anchor comprises, at its proximal end, an attaching and detaching mechanism for the attachment and detachment of a delivery element for the percutaneous deployment of said device.

In one exemplary embodiment, a fistula-free hemodialysis method disclosed herein consists of percutaneously deploying a first said intracorporeal conduit and anastomosing it to an artery for creating arterial access and a second said intracorporeal conduit and anastomosing it to a vein for creating venous access and independently connecting each of said devices respectively to the arterial and venous lines of said dialyzer using said transcutaneous ports.

The contributions of the present invention in addressing current hemodialysis needs with respect to prior art are manyfold. The devices disclosed in the present invention can be delivered percutaneously and without employing sutures and provide independent access to arteries and veins. The methods disclosed herein allow for the creation of fistula-free vascular access for hemodialysis without the need for an arteriovenous fistula, arteriovenous graft or central venous catheter. Thus, the risks and issues associated with the creation and maintenance of vascular access for hemodialysis are significantly reduced. Moreover, the devices disclosed herein provide access for required hemodialysis debits of 300-600 ml/min to and from blood vessels as small as 2-3 mm in diameter, thus increasing the number of choices for vascular access points in a patient's vasculature. Further, the devices and methods disclosed herein allow for a needleless connection between a patient and a dialyzer, thus simplifying and decreasing the burden of the hemodialysis procedure in hospitals and homecare settings.

### BRIEF DESCRIPTION OF THE DRAWINGS

FIG. **1** illustrates an example embodiment of a method of use of the present invention.
FIG. **2** illustrates an example embodiment of a transcutaneous access port according to the present invention.
FIG. **3** illustrates an example embodiment of a device anchor according to the present invention.
FIG. **4** illustrates an example embodiment of a device anchor connected to the distal end of an intracorporeal conduit according to the present invention.
FIG. **5** illustrates an example embodiment of a device anchor with a variable angle between said anchor body and its expandable segments.
FIG. **6** illustrates an example embodiment of an intracorporeal conduit deployed and attached to a blood vessel or graft using a device anchor according to the present invention.
FIG. **7** illustrates a superimposed view of an expanded and non-expanded example embodiment of a device anchor attached to a conduit according to the present invention.
FIG. **8A** illustrates a 3D view of an example embodiment of an expanded (pre-shaped or preprogrammed) device anchor according to the present invention.
FIG. **8B** illustrates 3D view of an example embodiment of a non-expanded device anchor according to the present invention.
FIG. **9A** illustrates a circular cross-sectional view of an expanded distal end of an example embodiment of a device anchor according to the present invention.
FIG. **9B** illustrates an elliptical cross-sectional view of an expanded distal end of another example embodiment of a device anchor according to the present invention.
FIG. **10** illustrates a 3D view of an example embodiment of a laser cut strut pattern for a device anchor in a non-expanded configuration according to the present invention.
FIG. **11** illustrates a 3D view of an example embodiment of a device anchor in an expanded configuration anastomosed to a blood vessel or graft according to the present invention.
FIG. **12** illustrates a lateral view of another non-expanded example embodiment laser cut strut pattern of a device anchor suited for deployment at an angle with respect to a target vessel according to the present invention.
FIG. **13** illustrates a frontal view of another non-expanded example embodiment laser cut strut pattern of a device anchor suited for deployment at an angle with respect to the target vessel according to the present invention.
FIG. **14** illustrates a superimposed view of an expanded (pre-shaped or preprogrammed) and non-expanded example embodiment laser cut strut pattern of a device anchor suited for deployment at an angle with respect to the target vessel according to the present invention.
FIG. **15** illustrates an example embodiment of the deployment of a device anchor at an angle with respect to a target vessel according to the present invention.
FIG. **16A** illustrates a 3D view of another example embodiment laser cut strut pattern of a device anchor in a non-expanded configuration that minimizes device weight and contact surface with the target conduit according to the present invention.
FIG. **16B** illustrates a 3D superimposed view of another example embodiment of an expanded (pre-shaped or preprogrammed) and non-expanded device anchor according to the present invention.
FIG. **17A** illustrates a 3D view of another example embodiment of an expanded (pre-shaped or preprogrammed) device anchor attached to the distal segment of an intracorporeal conduit according to the present invention.
FIG. **17B** illustrates a cross-sectional view of the device illustrated at FIG. **17A****.**
FIG. **18A** illustrates a 3D view of an example embodiment laser cut strut pattern of a device anchor with a female-side bayonet attaching mechanism in a non-expanded configuration according to the present invention.
FIG. **18B** illustrates a 3D view of an example embodiment of an expanded (pre-shaped or preprogrammed) device anchor with a female-side bayonet attaching mechanism, said anchor being attached to a distal segment of an intracorporeal conduit according to the present invention.
FIG. **19A** illustrates a lateral view of an example embodiment of a delivery element with a male-side bayonet attaching mechanism according to the present invention.
FIG. **19B** illustrates a longitudinal sectional view of an example embodiment of a delivery element with a male-side bayonet attaching mechanism according to the present invention.
FIG. **19C** illustrates a cross-sectional view of an example embodiment of a delivery element with a male-side bayonet attaching mechanism according to the present invention.
FIG. **20** illustrates an example embodiment of a method of loading a device anchor and intracorporeal conduit into a loader using a delivery element according to the present invention.
FIG. **21** illustrates an example embodiment of a device deployment kit for percutaneous and sutureless deployment of an intracorporeal conduit and device anchor according to the present invention.
FIG. **22** illustrates an example embodiment of a method for percutaneous and sutureless attachment of an intracorporeal conduit to a blood vessel or graft using a device anchor and a device deployment kit according to the present invention.
FIG. 23 illustrates an example embodiment of a device and method for needlelessly connecting a patient to a dialyzer or to another external device according to the present invention.
FIG. 24 illustrates another example embodiment of a transcutaneous access port placed subcutaneously according to the present invention.
FIG. 25 illustrates another example embodiment of a transcutaneous access port placed subcutaneously and comprising an adaptor for several predefined needle access points (buttonholes) according to the present invention.

### DETAILED DESCRIPTION

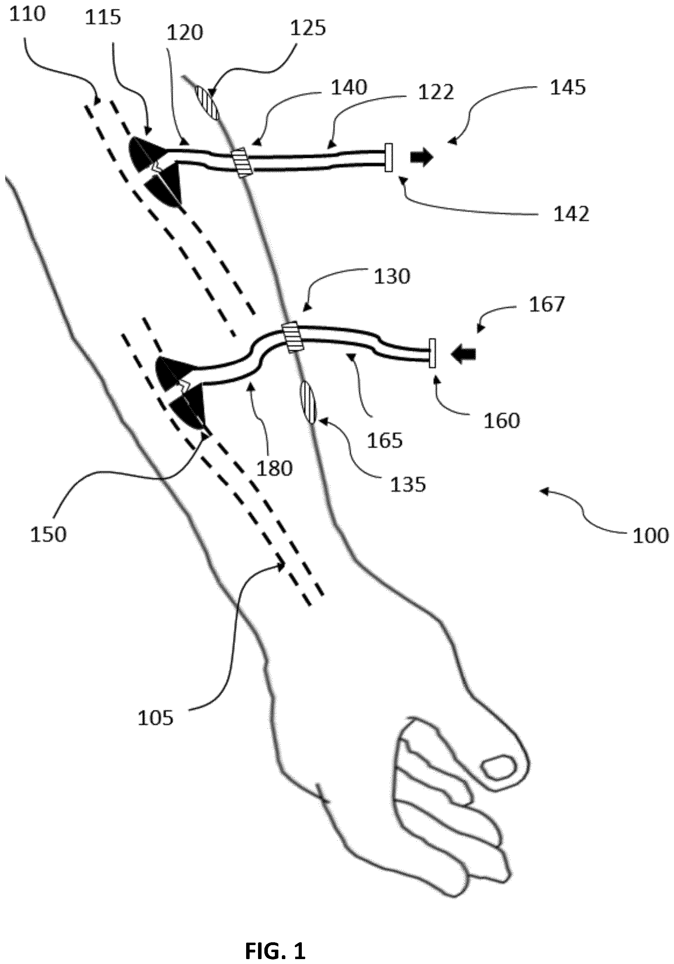

FIG. **1** illustrates an exemplary context of use **100** of the present invention. In order to create vascular access for hemodialysis, a first intracorporeal conduit **120** is deployed percutaneously through a skin area **125** and attached to the wall of a conduit in the body, e.g., to the wall of a blood vessel or graft **110** using the device anchor **115** as described further herein. In one embodiment of the present invention, blood vessel **110** is an artery. In one embodiment of the present invention, the conduit **120** is attached to transcutaneous access port **140** in order to facilitate the connection of conduit **120** to an external device using conduit **122** and connector **142.** In one embodiment of the present invention, transcutaneous port **140** is attached to the patient's skin. In one embodiment of the present invention, conduit **122** can be attached to and detached from transcutaneous access port **140.** In one embodiment of the present invention, connector **142** is attached to the arterial line of a hemodialysis machine (dialyzer) and blood can be drawn (**145**) from artery **110** into the dialyzer during the hemodialysis procedure.

A second intracorporeal conduit **180** is deployed percutaneously through a skin area **135** and attached to the wall of a conduit in the body, e.g., to the wall of a blood vessel or graft **105** using the device anchor **150** as described further herein. In one embodiment of the present invention blood vessel **105** is a vein. In one embodiment of the present invention, conduit **180** is attached to transcutaneous port **130** in order to facilitate the connection of conduit **180** to an external device using conduit **165** and connector **160.** In one embodiment of the present invention, transcutaneous access port **130** is attached to the patient's skin. In one embodiment of the present invention, conduit **165** can be attached to and detached from transcutaneous access port **130.** In one embodiment of the present invention, connector **160** is attached to the venous line of a hemodialysis machine (dialyzer) and blood can be infused (returned) (**167**) into vein **105** from the dialyzer during the hemodialysis procedure.

In another embodiment of the present invention, a first and a second intracorporeal conduits can be attached (anastomosed) at different locations on the same conduit in the body, e.g., on the same vein or a graft.

In another embodiment of the present invention, only one intracorporeal conduit and transcutaneous port is deployed, e.g., to a vein or graft for single needle hemodialysis.

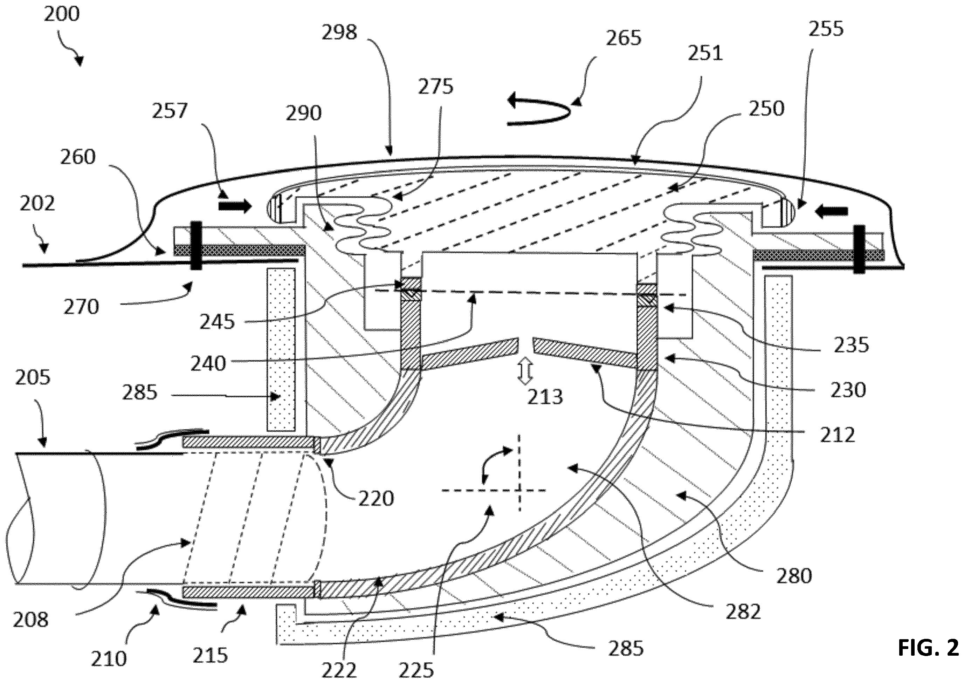

FIG. **2** illustrates an example embodiment of a transcutaneous access port **200** outside the subject-matter of the claims but useful for understanding the present invention. Said port allows for repetitive needles access to an intracorporeal conduit **205** in a patient's body through the patient's skin **202.** A subcutaneous segment **215** of port **200** can be connected to the proximal end of said conduit **205** as described herein. The distal end of conduit **205** can be indwelling in a patient's blood vessel or anastomosed to another conduit of the human body, e.g., to a blood vessel or to an arteriovenous graft.

As illustrated by the embodiment in FIG.**2** outside the subject-matter of the claims but useful for understanding the present invention, an intracorporeal conduit according to the present invention can be connected to the port body **280** by pushing the conduit **205** into the segment **215** of the port body. The conduit **205** can also be pushed over the segment **215** of the port body. The conduit **205** and the inner wall of the segment **215** can be configured with matching threads **208**, such that conduit **205** can be threaded into the segment **215.** The conduit **205** is pushed or threaded into the segment **215** up to a stop **220.** The width of stop **220** matches the wall size of conduit **205** such that the inner lumen presents no obstacle to the flow through the conduit and laminar flow is ensured. After inserting the conduit **205** into or over segment **215** of the port body, a band or ring **210** is deployed to additionally secure in place the connection between the conduit **205** and the port body segment **215.**

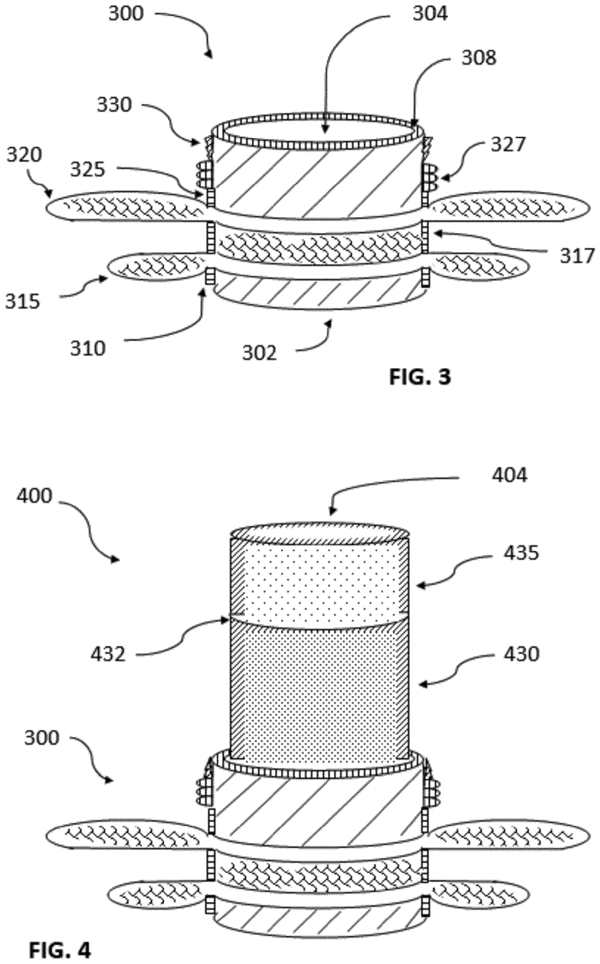

FIG. **3** illustrates an example embodiment of a tubular device anchor according to the present invention. Device **300** comprises a body **302** having an inner lumen **304**, an expandable distal segment **315**, an expandable waist segment **317,** an expandable proximal segment **320,** and attachment segments **327** and **330.**

In one embodiment of the present invention, distal segment **315** self-expands in the lumen of a conduit, e.g. a blood vessel or graft and abuts the conduit wall from the inside of the conduit. In one embodiment of the present invention, proximal segment **320** self-expands outside the conduit and abuts said conduit wall from the outside of the conduit. In one embodiment of the present invention, waist segment **317** has a width approximately equal to the thickness of the blood vessel or graft wall, connects said distal **315** and proximal **320** segments of the device and ensures that the distal and proximal segments are applying the appropriate amount of pressure to said conduit wall as to keep the device steady on the conduit wall.

In one embodiment of the present invention, the proximal segment **320** is larger in size than the distal segment **315** in order to provide a larger footprint outside the blood vessel and a smaller footprint inside the blood vessel. The larger footprint outside the blood vessel provides better protection and more reliable attachment to the blood vessel. The smaller footprint inside the blood vessel reduces turbulences in flow and the risks associated with thrombosis and vessel wall damage, e.g., stenosis.

Distal segment **315** and proximal segment **320** of device **300** can be made of medical-grade stainless steel or of cobalt alloy or of a shape memory alloy, e.g., nitinol or of other shape memory materials, such as shape memory polymers, or of other superelastic material.

In one embodiment of the present invention, said distal and proximal segments, as well as the waist segment, are appropriately sized pieces of alloy fabric. One can solder, braze, weld or otherwise affix the ends of the braided alloy fabric to a distal ring **310**, to waist **317**, if the case, and to a proximal ring **325.**

In one embodiment of the present invention, said distal and proximal segments are laser cut struts in a tube of shape memory material and pre-shaped or preprogrammed in the desired configuration as described further herein. In one embodiment of the present invention, said device is formed by laser cutting patterns in shape memory material tubing or sheets that are subsequently rolled into cylinders whose edges are welded together.

In one embodiment of this invention, the wall thickness **308** of the tubular device structure is in the range of 0.1 mm in order to allow for deployment in small peripheral blood vessels. In another embodiment of the present invention, each or all of the said segments of the device **300** or device **300** in its entirety can be printed using multi-material 3D and 4D printing technologies.

In one embodiment of the present invention, distal segment **315** is drug coated in order to minimize the damage to the vessel wall and prevent stenosis and thrombosis, e.g., with paclitaxel or sirolimus. In another embodiment of the present invention, said distal segment is gold plated in order to lower thrombogenicity.

In one embodiment of the present invention, attachment segment **327** is used to attach a delivery element to device **300** according to the present invention. In one embodiment of the present invention, a delivery element is attached to device **300** by screwing it onto the attachment threads **327** and is detached from the said device by unscrewing it. In another embodiment of the present invention, the attachment mechanism **327** can be a "bayonet" lock.

In one embodiment of the present invention, attachment segment **330** is used to attach device **300** to the distal end of a conduit, e.g., arteriovenous graft or arteriovenous shunt. In one embodiment of the present invention the conduit is attached to the device **300** by force pushing the end of the conduit over the attachment segment and securing it in place using heat shrink tubing. In another embodiment of the present invention, attachment mechanism **330** can be a "bayonet" lock or a threaded screw-unscrew attachment. In another embodiment of the present invention, attachment mechanism **330** can be made to match an appropriate segment of the graft or shunt end to which it is connected.

In one embodiment of the present invention, attachment segments **327** and **330** can be made of the same material as the any of the other segments of device **300** or of stainless steel, silicone, PTFE, polyurethane or of any other suitable biocompatible material.

In one embodiment of the present invention, device anchor **300** can have a circular cross-section. In another embodiment of the present invention, the device anchor **300** can have an elliptical cross-section, in which case the longer axis of such ellipsis must be aligned with the longitudinal axis of the target conduit.

In one embodiment of the present invention, the inner cross-section of the device **300** is of such size as to ensure the appropriate debit of fluid required for a hemodialysis procedure, e.g., in the range of 300 - 600 ml /sec.

In one embodiment of the present invention, device **300** or at least one of its segments is radiopaque and/or echogenic. In one embodiment of the present invention, device **300** is MRI compatible, power injectable and Latex, DEHP and PVC free. In one embodiment of the present invention, device **300** is marked such as to allow an easy deployment under ultrasound guidance and an easy alignment along the blood vessel in the case of an elliptical embodiment.

FIG. **4** illustrates one example embodiment of a device according to the present invention. Device **400** consists of a device anchor **300** and an intracorporeal conduit **404.** In one embodiment of the present invention, conduit **404** extends into the anchor **300** to the distal end of said anchor such that the distal end of said conduit and the distal end of said anchor are flush. In one embodiment of the present invention, the distal end of intracorporeal conduit **404** is fixedly attached to the distal end of device anchor **300.** In another embodiment of the present invention, the distal end of intracorporeal conduit **404** is fixedly attached to waist **317** of device anchor **300.**

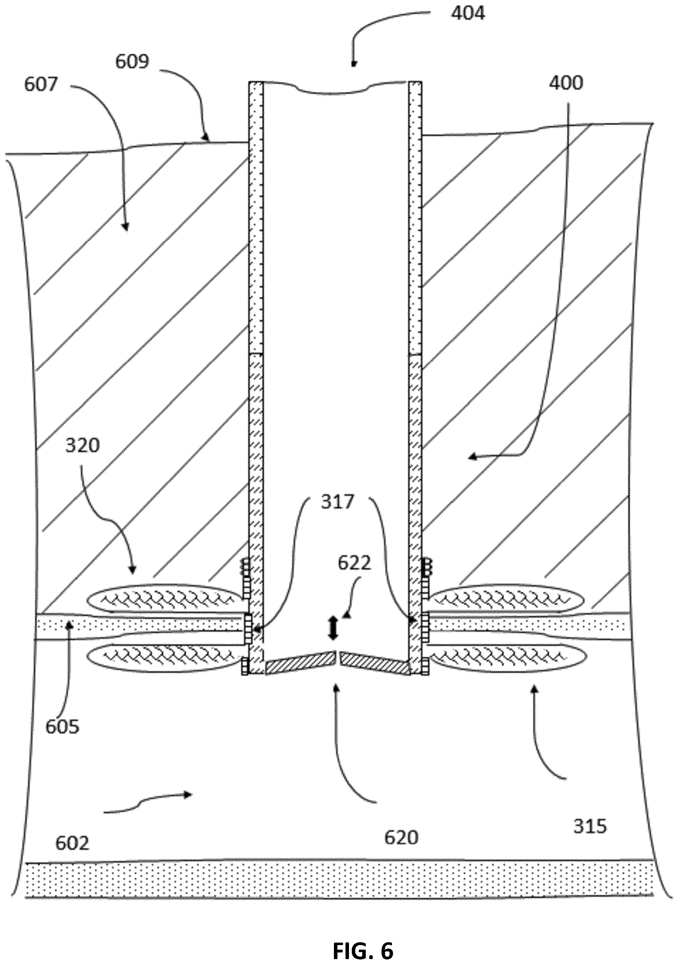

In one embodiment of the present invention, the conduit **404** comprises a 3-way (infusion, aspiration, and closed) valve, as illustrated at **620** on FIG. **6****.** The valve minimizes the risk of conduit blockage due to thrombosis by still allows for easy infusion and aspiration. In another embodiment of the present invention, the conduit **404** extends only partially into anchor **300** and a three-way valve is built into the distal end of said anchor **300** at the level of the distal ring **310.** In one embodiment of the present invention, conduit **404** and valve **620** are made from the same material, e.g., silicone, PTFE, polyurethane. In one embodiment of the present invention said conduit and valve can be 3D or 4D printed.

In one embodiment of the present invention, the device can be built in different sizes, such as to allow deployment in larger vessel, e.g., in the range of 6 mm in diameter in the upper arm or upper legs and in smaller vessels, for example in blood vessels in the range of 2 mm diameter corresponding to arteries and veins of the lower arm or lower leg. Further, the sizes of the device are such as to allow deployment at deeper depths, e.g., in the range of 6 cm in the upper arm and leg and at shallower depths, for example in the range of 2 cm in the lower arm and leg.

In one embodiment of the present invention, after the percutaneous deployment and attachment of device **400** to the wall of a blood vessel or graft as further described herein, the proximal end of conduit **404** remains out of the patient's body such that it can be connected to another device, as described further herein. In one embodiment of the present invention the said conduit is trimmable and marked with cm markings.

In one embodiment of the present invention, conduit **404** is made of two segments **430** and **435** of different materials with different properties. In one embodiment of the present invention, segment **430** extends from the distal end of the conduit (and of anchor 300) for a minimal length equal to the length of anchor **300** when said anchor is in a compressed (non-expanded) configuration as illustrated and described in FIG. **6****.**

In one embodiment of the present invention, segment **430** is made of such material as to be more rigid than segment **435** in order to allow for the proximal segment of anchor **300** to easily slide over said more rigid segment **430** during the deployment of the device. In one embodiment of the present invention, segment **430** is made of such material as to allow for reliable attachment of anchor **300** to conduit segment **430** at their respective distal ends. In one embodiment of the present invention, segment **430** can be made of silicone, PTFE, polyurethane or similar materials of different rigidity than segment **435** and/or braided with braids made of stainless steel or nitinol or similar alloys. In one embodiment of the present invention, segment **430** is made of titanium in order to minimize blood clotting. In one embodiment of the present invention, segment **430** is gold plated in order to lower thrombogenicity. Segments **430** and **435** can be attached together using heat shrink tubing, overmolding or co-extrusion, by using an attachment mechanism **330**, by pushing segment **435** over or into segment **430** or segment **430** can be made by additionally braiding and hardening segment **435.**

In one embodiment of the present invention, the ratio between the outer diameter of said device anchor **300** in a compressed (non-expanded) configuration and the inner diameter of the more rigid distal segment **430** of conduit **404** is minimized by minimizing the wall thickness of said device anchor and the wall thickness of said more rigid distal segment such as to allow for fluid debits required for hemodialysis in the range of 300-600 ml/min while allowing attachment to small peripheral veins and arteries in the range of 2-3 mm in diameter.

In one embodiment of the present invention, segment **435** extends from the proximal end **432** of segment **430** to the proximal end of conduit **404** outside the patient's body. In one embodiment of the present invention, segment **435** is made of silicone, PTFE, polyurethane, or similar materials.

In one embodiment of the present invention, device **400** can be removed from the vessel wall and from the patient's body percutaneously through a procedural sheath according to the present invention. The opening left in the vessel wall after removal can be closed through the procedural sheath or over a guidewire using a standard vessel closure device. In one embodiment of the present invention, a method for percutaneously removing said device from the wall of a blood vessel comprises the following steps:
1. Detaching the proximal end of the intracorporeal conduit from the transcutaneous port, if attached.
2. Inserting a guidewire through the intracorporeal conduit into the target blood vessel.
3. Inserting a delivery element over the intracorporeal conduit until the distal end of the delivery element reaches the proximal end of the device anchor.
4. Inserting a delivery sheath over the delivery element until the delivery sheath reaches the outside of the blood vessel wall.
5. Under ultrasound imaging guidance, attaching the attaching mechanism of the delivery element to the attaching mechanism of the device anchor.
6. Pulling the delivery element thus pulling the device anchor and the distal end of the intracorporeal conduit into the delivery sheath while pushing the delivery sheath into the opening left in the wall of the blood vessel or graft by the device anchor.
7. After the delivery sheath has been pushed into the blood vessel or graft, pulling back the delivery element thus pulling back and out of the body the device anchor and the intracorporeal conduit through the delivery sheath.
8. Closing the wall opening in the wall of the blood vessel or graft through the delivery sheath and over the guidewire using a closure device.

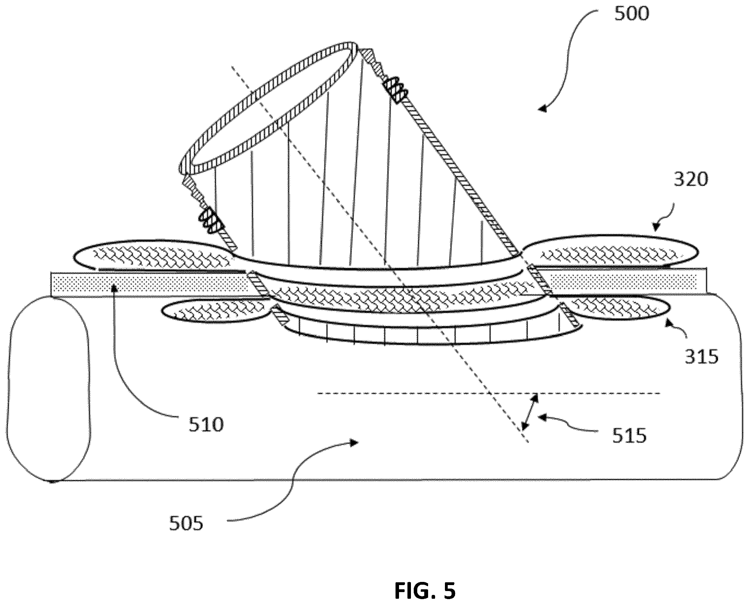

FIG. **5** illustrates an example embodiment of a device anchor **500** with a variable angle **515** between said anchor body and its expandable segments **315** and **320.** Device anchor **500** can be attached to the wall **510** of a blood vessel or a graft **505** such that an appropriate value for angle **515** between the longitudinal axis of said blood vessel or graft **505** and the longitudinal axis of the inner lumen of said device **500** can be chosen in order to provide optimized fluid dynamics though the device relative to the location of the device attachment and to the desired path of attached intracorporeal conduit.

FIG. **6** illustrates an example embodiment of a device **400** deployed in a blood vessel or a graft **602** through patient skin **609** and surrounding tissue **607** according to the present invention. The distal segment **315** of device **400** is placed in the lumen **602** of the blood vessel, apposed from the inside to the inner surface of the wall **605** of the target conduit, e.g., a blood vessel. The proximal segment **320** of device **400** is placed outside the vessel, apposed from the outside to the outer surface of the wall **605** of said target conduit. Waist segment **317** of device **400** connects the distal and proximal segments of the device and ensures that the said segments are applying the right amount of the pressure to the vessel wall **605** as to keep the device steady in place. The conduit **404** of device **400** exits the patient skin and can be trimmed and attached to an external device.

In one embodiment of the present invention, the 3-way (**622**) (infusion, aspiration, and closed) valve **620** minimizes risk of conduit blockage due to thrombosis by allowing infusion and aspiration of fluid to and from target conduit **602** through device conduit **404** only upon demand and by preventing fluid, e.g., blood from entering conduit **404** when the device is not used for infusion or aspiration.

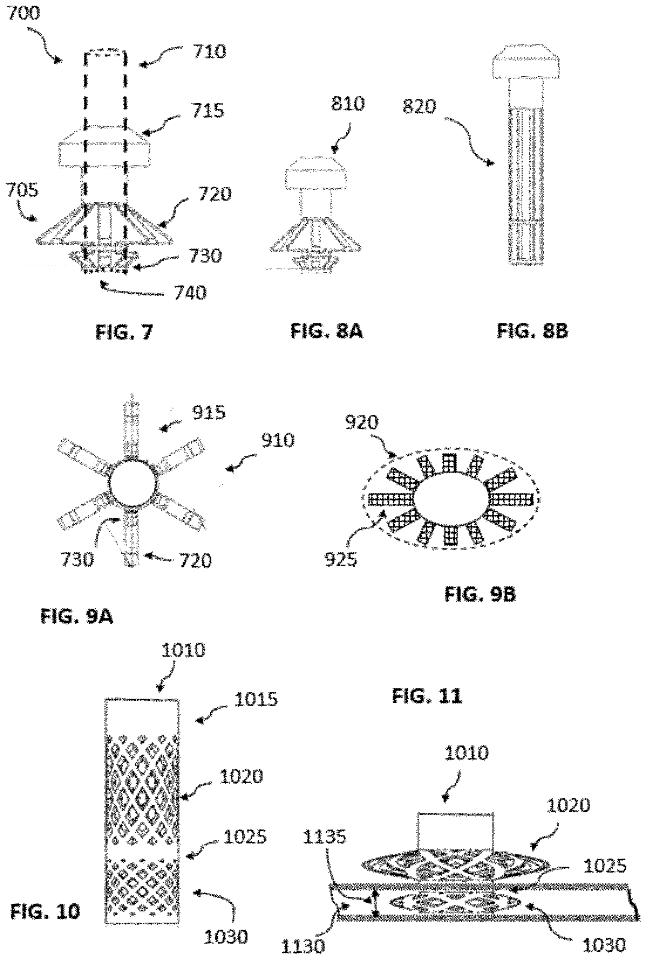

FIG. **7** illustrates a superimposed view **700** of another embodiment of an expanded (pre-shaped) and non-expanded device anchor **705** attached to the distal segment of intracorporeal conduit **710** comprising distal valve **740.** A delivery element can be attached to and detached from said device anchor by using attaching and detaching mechanism **715.** Proximal expandable segment **720** and distal expandable segment **730** are configured as expandable struts, wherein the struts width is optimized to apply ensure pressure on the target vessel wall and to minimize the device weight and contact surface with said vessel wall, and wherein the proximal segment **720** is larger in size than the distal segment **730** in order to ensure reliable attachment to the vessel wall while minimizing the amount of material present within the vessel, thus minimizing the risk of thrombosis and stenosis.

FIG. **8A** illustrates a 3D view of device anchor **705** in an expanded (pre-shaped) configuration with struts cut from a tube of shape memory material **810** after shape setting of said material according to the present invention.

FIG. **8B** illustrates a 3D view of device anchor **705** in a non-expanded configuration with struts cut from a tube of shape memory material **820** before shape setting of said material according to the present invention.

In one embodiment of the present invention, device anchor **705** is a thin walled cylindrical structure, wherein the distal, waist and proximal segments of said device are formed using a shape memory material, said distal and proximal segments consisting of several longitudinal struts disposed around the circumference of said cylindrical structure, said waist segment being ring-shaped, wherein said longitudinal struts of said proximal segment are attached at their distal end to said waist ring, wherein said longitudinal struts of said proximal segment are attached to a second ring at their proximal end, wherein said longitudinal struts of said distal segment are attached at their proximal end to said waist ring, wherein the distal and the proximal segments have the same or a different number of struts and, wherein the struts have the same or different widths, and wherein the struts are disposed equidistantly or at variable distance from each other around the circumference of said cylindrical structure **820.**

In one embodiment of the present invention, said struts of said distal and proximal segments are pre-shaped through shape setting in their radially expanded and longitudinally compressed configuration, wherein each longitudinal struts of the proximal segment is bent outwardly as to be set in a V or U-shape, wherein the two ends of each of the V or U-shaped struts are attached to the proximal ring and to the waist ring respectively.

In one embodiment of the present invention, said struts of said proximal segment are coupled to each other by structures extending generally laterally.

FIG. **9A** illustrates an example embodiment of a circular cross-sectional view **910** of an expanded distal end of device anchor **705** according to the present invention. The struts **915** are rectangular and disposed regularly in a circular pattern around a circular inner lumen. The struts of proximal segment **720** are of equal lengths. The struts of the distal segment **730** are of equal lengths. However, the struts of the proximal segment **720** are longer than the struts of the distal segment **730** in order to provide a larger surface for attachment to the vessel or graft wall.

FIG. **9B** illustrates another example embodiment of an elliptical cross-sectional view **920** of the expanded distal end of a device anchor according to the present invention. The struts **925** are rectangular, of different lengths as to provide an elliptical cross-section and disposed around an inner lumen of elliptical cross-section for better alignment with the longitudinal axis of the target vessel and thus allowing deployment in smaller vessels.

FIG. **10** illustrates a frontal view of an example embodiment laser cut strut pattern of a device anchor **1010** in a non-expanded configuration before shape setting according to the present invention. A tube **1015** of shape memory and/or superelastic material is cut using pattern 1020 for the proximal segment and a different pattern **1030** for the distal segment of said device anchor. The waist segment **1025** separates the distal segment **1030** from the proximal segment 1020.

FIG. **11** illustrates a 3D configuration view of an example embodiment of the laser cut strut pattern of device **1010** in an expanded and deployed configuration according to the present invention. The expanded proximal segment **1020** is deployed outside the blood vessel or graft wall and the expanded distal segment **1030** is deployed inside the blood vessel or graft in such a way as to expand over the whole inner lumen of said blood vessel or graft with blood flowing through the struts of expanded distal segment **1030.** The length **1135** of the expanded distal segment inside the blood vessel equals the inner diameter of blood vessel or graft **1130.** The waist segment **1025** of approximately the height of the thickness of the vessel or graft wall attaches the deployed device **1010** to said vessel wall, such that expanded proximal segment **1020** and expanded distal segment **1030** apply sufficient pressure on the vessel wall to keep said device **1100** steady in place.

In one embodiment of the present invention, the distal segment of said device comprises a plurality of struts forming a net-like structure **1030** that, when pre-shaped, expands radially and compresses longitudinally.

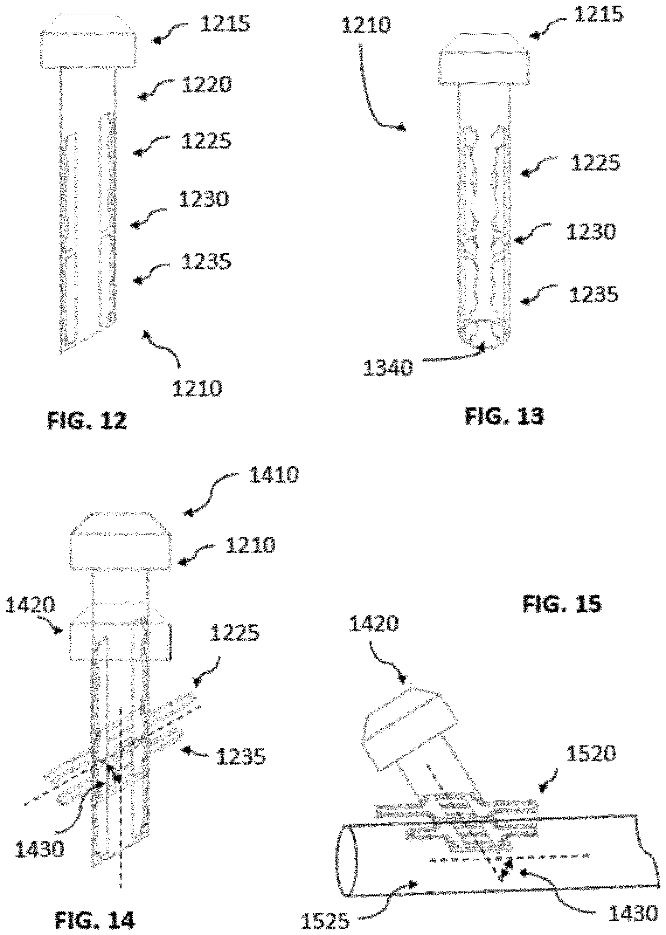

FIG. **12** illustrates a lateral view of yet another non-expanded example embodiment laser cut strut pattern of a device anchor **1210** suited for deployment at an angle with respect to the target vessel according to the present invention. A tube of shape memory and/or superelastic material **1220** is cut using certain patterns **1225** for the proximal segment, certain patterns **1230** for the waist segment, and certain patterns **1235** for the distal segment of a device anchor. The attaching mechanism **1215** can be cut from or welded to the same tube.

FIG. **13** illustrates a frontal view of device anchor **1210.** The inner lumen **1340** of the device anchor is used to introduce a matching intracorporeal conduit and attaching the distal end of said conduit to the distal end or to the waist segment of said device anchor. The different laser cut patterns of proximal segment **1225,** waist segment **1230**, and distal segment **1235** allow to obtain different shapes and configurations of said device anchor through shape setting of the shape memory tube **1220.** In one embodiment of the present invention, longitudinal struts of the distal and proximal segments are of variable width profiles that determine preferred bend locations of the struts for shape setting of the pre-shaped configuration of said device anchor. In one embodiment of the present invention, the plane of the waist ring **1230** is at a variable angle with respect to the cross-sectional plane of said cylindrical structure of said device anchor, and the plane of the distal end of the distal segment **1235** of said device anchor is parallel to the plane of the waist ring at said variable angle with respect to the cross-sectional plane of said device anchor

FIG. **14** illustrates a superimposed view **1410** of a non-expanded and expanded (pre-shaped) **1420** laser cut strut pattern of device anchor **1210** suited for deployment at angle **1430** with respect to the target vessel according to the present invention. In one embodiment of the present invention, segments **1225** and/or **1235** are double or multiple flipped.

FIG. **15** illustrates an example embodiment of device anchor **1210** deployed at angle **1430** with respect to the target vessel **1525** according to the present invention. The fixation distal and proximal segments **1520** are parallel to the vessel wall, while the longitudinal axis of the deployed device anchor **1420** are at angle **1430** with respect to the longitudinal axis of said target vessel **1525.**

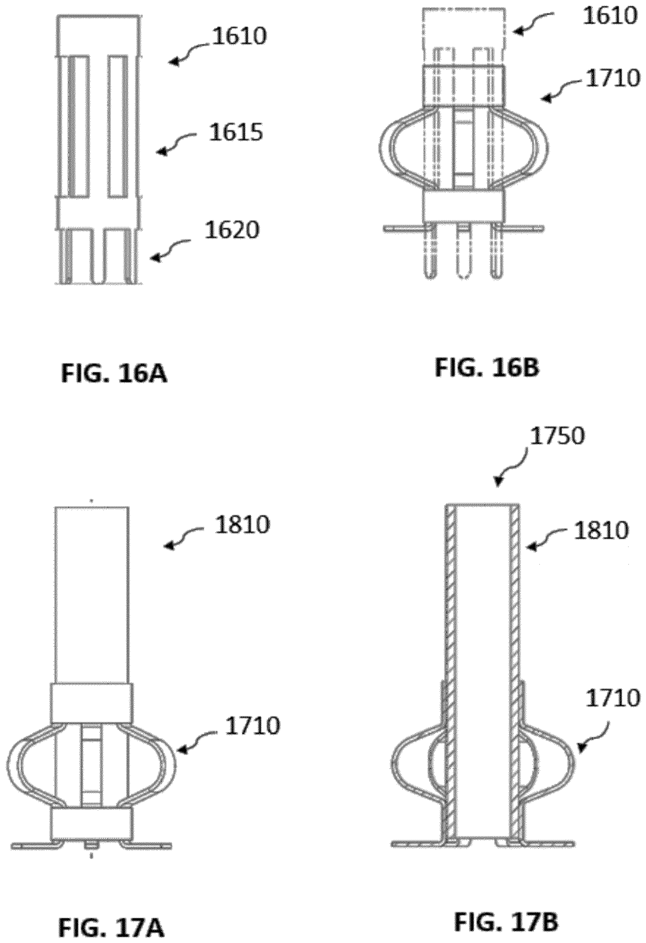

FIG. **16A** illustrates a 3D view of another example embodiment laser cut strut pattern of a device anchor in a non-expanded configuration **1610** according to the present invention. The distal segment **1620** of said device anchor presents an open strut pattern that minimizes the volume of the material of the distal segment of the device anchor present in the blood vessel after deployment. In one embodiment of the present invention, proximal segment **1615** has a different number of struts than distal segment **1620.** In one embodiment of the present invention, the struts of the proximal segment **1615** have a different width than the struts of the distal **1620.** In one embodiment of the present invention, the struts of the distal segment **1620** have different lengths **925** in order to implement the elliptical shape **920** on FIG. **9B****.**

FIG. **16B** illustrates a superimposed view of a non-expanded **1610** and expanded (pre-shaped) **1710** laser cut strut pattern of device anchor at FIG. **16A** according to the present invention. When expanded, the longitudinal struts of the distal segment extend outwardly and perpendicularly to the longitudinal axis of the inner lumen of said device, as to minimize the contact surface between said struts and the inner vessel wall and the blood in the vessel and as to minimize the device weight.

FIG. **17A** illustrates a 3D view of another example embodiment of an expanded (pre-shaped) device anchor **1710** attached to the distal segment **1810** of an intracorporeal conduit according to the present invention.

FIG. **17B** illustrates a cross-sectional view **1750** of the expanded (pre-shaped) device anchor **1710** attached to the distal segment of the intracorporeal conduit **1810** as illustrated at FIG. **17A****.**

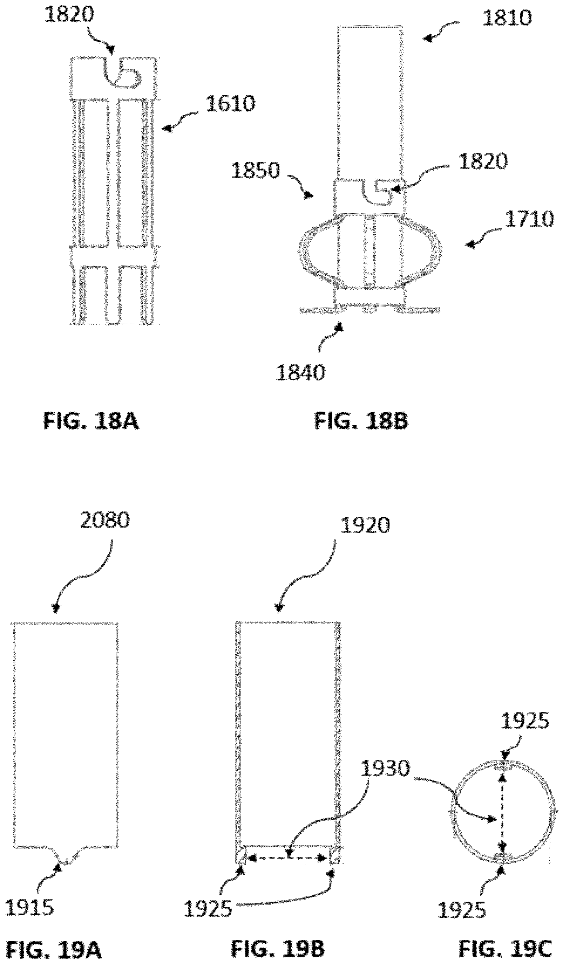

FIG. **18A** illustrates a 3D view of an example embodiment laser cut strut pattern of a device anchor in a non-expanded configuration **1610** with a female-side bayonet attaching mechanism (bayonet mount or bayonet connector) **1820** according to the present invention.

FIG. **18B** illustrates a 3D view of an example embodiment of the expanded (pre-shaped) device anchor **1710** with a female-side bayonet attaching mechanism (bayonet mount or bayonet connector) **1820**, said device anchor being attached to the distal end **1840** of the intracorporeal conduit **1810** according to the present invention. The proximal end **1850** of the device anchor can slide over the distal segment **1810** of an intracorporeal conduit according to the present invention.

FIG. **19A** illustrates a lateral view of an example embodiment of a tubular delivery element **2080** with a male-side bayonet attaching mechanism **1915** with male side pins oriented inwards according to the present invention. The male-side bayonet attaching mechanism **1915** matches the bayonet female-side attachment mechanism **1820** of the device anchor **1710.**

FIG. **19B** illustrates a longitudinal section **1920** of an example embodiment of a tubular delivery element **2080** with a male-side bayonet attaching mechanism **1915** according to the present invention. Pins **1925** of the male-side bayonet mechanism are interior to the tubular delivery element **2080** such that the distance **1930** between the inner edges of the bayonet pins is equal or slightly larger than the outer diameter of distal segment **1810** of the intracorporeal conduit such as to reliably engage the matching bayonet attaching mechanism **1820** of said device anchor and to slide over the distal segment of the intracorporeal conduit **1810.** After attaching the bayonet connector **1915** of the delivery element, i.e., its respective pins **1925** to the corresponding bayonet connector **1820** of the device anchor **1710**, the delivery element **2080** can be used to pull the proximal end of the device anchor **1710** along the distal segment of the intracorporeal conduit **1810** as described further herein.

FIG. **19C** illustrates a cross-sectional view of an example embodiment of a tubular delivery element with a bayonet attaching mechanism according to the present invention, whereby the pins **1925** of the bayonet connector are internal and oriented inwards to the delivery element in order to be able to attach to the matching bayonet connector **1820.**

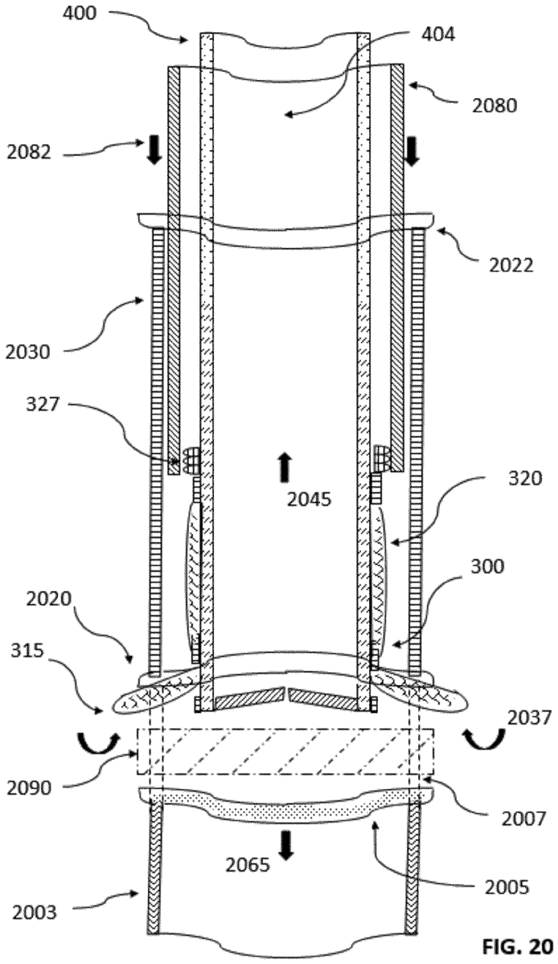

FIG. **20** illustrates an example embodiment of a method of introducing device **400** into delivery sheath **2003** by using loader **2030** and delivery element **2080** according to the present invention. In one embodiment of the present invention, loader **2030** is a tubular structure with female luer connectors at both its distal **2020** and proximal ends **2022.** At the distal end, female luer connector **2020** of loader **2030** can be connected to female luer connector **2005** of delivery sheath **2003** using a male-to-male luer connector **2090.** The connection is realized such as to ensure alignment **2007** between the inner wall of the loader **2030** and the inner wall of the lumen of the delivery sheath **2003.**

In one embodiment of the present invention, the delivery sheath **2003** can be connected at its proximal end to a hemostasis valve **2170.** In such case, the male-to-male luer connector **2090** connects the female luer connector **2020** of loader **2030** to the female luer connector of hemostasis valve **2170** and device **400** is transferred from loader **2030** to delivery sheath **2003** through the connector **2090** and through the hemostasis valve.

In one embodiment of the present invention, delivery element **2080** is a tubular structure, with an attachment mechanism at its distal end that can be attached to the matching attaching mechanism **327** of device **400.** The delivery element is made of such materials as to be flexible enough and to provide enough torque and push/pull strength in order to allow for the use described herein.

The loader **2030,** delivery element **2080,** and the delivery sheath **2003** can be made of materials such as silicone, PTFE, polyurethane or other similar materials. In a preferred embodiment according to the present invention, loader **2030** is transparent in order to be able to visualize that device **400** is properly loaded.

In one embodiment of the present invention, loader **2030,** delivery element **2080,** and/or delivery sheath **2003** can be made using 3D and 4D printing of appropriate biocompatible materials.

In one embodiment of the present invention, a method of loading the device **400** into loader **2030** and deploying it in a blood vessel through a delivery sheath **2003** consists of the following steps:
1. The distal end of delivery element **2080** is inserted into a peel-away loader **2030** at the loader's proximal end and is pushed towards the loader's distal end such that the distal end of the said delivery element exits the distal end of said loader. The proximal end of said delivery element remains outside the proximal end of said loader.
2. The proximal end of intracorporeal conduit **404** is passed through delivery element **2080** (that has previously been inserted into the loader **2030**) starting at the distal end of the said delivery element, such that device anchor **300** of device **400** remains outside the distal end of the said delivery element **2080.** The proximal end of said intracorporeal conduit **404** exits the delivery element through the proximal end of said delivery element.
3. The distal end of the delivery element **2080** is attached to device anchor **300** using the attaching mechanism **327.**
4. Device **400** is pulled back (**2045**) into the loader **2030** by pulling back the delivery element **2080** such that the device anchor **300** is pulled back and compressed in the inner lumen of loader **2030.** FIG. **20** illustrates an intermediate step of device loading according to the present invention wherein the proximal segment **320** of device anchor **300** of device **400** is compressed inside the loader while the distal segment **315** of the device anchor **300** of device **400** is in the process of flipping **(2037)** and being compressed into the loader.
5. After device **400** is fully loaded into loader **2030** and after the delivery sheath **2003** has been properly placed in the patient's body in the target vessel, the distal end of said loader is connected to said delivery sheath using a female luer lock to female luer lock connector **2090.**
6. Device **400** is then fully pushed (**2065**, **2082**) into the delivery sheath **2003** by pushing the delivery element **2080** and, after the distal end of device **400** has been completely inserted in said delivery sheath, the loader **2030** is disconnected and pulled back or peeled away and removed.
7. Device **400** is placed in the vessel wall using the delivery sheath **2003** and the delivery element **2080** by a combination of pushing said delivery element and pulling back said delivery sheath.
8. After verification by ultrasound imaging of proper placement of device **400** in the vessel wall, delivery element **2080** is detached from the attaching mechanism **327** and pulled back out of the delivery sheath **2003** or peeled away and removed.
9. Finally, the delivery sheath **2003** is pulled back or peeled away and removed.
10. Through the entire deployment procedure, a hemostasis valve **2170** with extension tube **2175** and stopcock **2180** can be connected to the female luer lock **2005** of the delivery sheath **2003** and can be used as required for flushing the delivery system and controlling back-bleeding.

In another embodiment of the present invention, steps 5, 6, and 7 of the above described method of loading the device **400** into loader **2030** and deploying it in a blood vessel through a delivery sheath **2003** are replaced by the following steps:
1. After device **400** is fully loaded into loader **2030** and after the delivery sheath **2003** has been properly placed in the patient's body in the target vessel, the distal end of said loader is pushed into the delivery sheath by pushing the proximal end of said loader until the distal end of the loader, which is flush with the distal end of the intracorporeal conduit, has reached the distal end of the delivery sheath.
2. Attaching the intracorporeal conduit of claim **1** to the vessel wall by allowing the device anchor of claim **1** to self-expand by a combination of pushing said delivery element and pulling back said loader.
3. Pulling back and/or peeling away the loader.

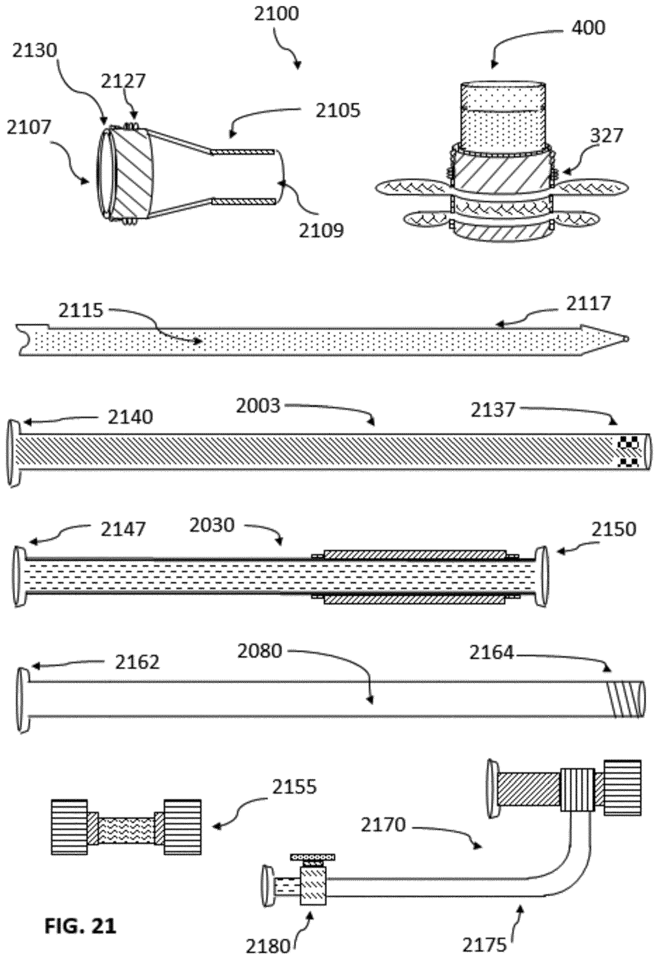

FIG. **21** illustrates an example embodiment of a deployment kit for percutaneous and sutureless deployment of device **400** according to the present invention. In one embodiment of the present invention, the kit **2100** consists of:
1. Implantable device **400.**
2. Device-graft adapter **2105** used to adapt the size and/or shape of device **400** to a potentially different size and/or shape of a connected graft or shunt. Device **400** is connected at the distal end **2109** of adaptor **2105,** while the graft or shunt is connected to the proximal end **2107** of adaptor **2105.** Adaptor **2105** comprises an attachment segment **2127** for attaching a delivery element and segment **2130** for attaching said adaptor to a graft or shunt. Adaptor **2105** can be made of silicone, PTFE, polyurethane, nitinol, titanium or other similar materials and can be overmolded and/or co-extruded using a combination of such materials.
3. Dilator/introducer **2115** with a blood flow back path **2117** for accurate positioning of delivery sheath **2003** at the blood vessel wall as described herein and wherein said dilator/introducer can be deployed into the target vessel over a procedural guidewire.
4. Peel-away delivery sheath **2003** with a female luer connector **2140** at the proximal end. In one embodiment of the present invention, delivery sheath **2003** has echogenic orientation markers **2137** at the distal end for identification of the long axis of an elliptical cross-section and orientation along the longitudinal axis of the targe blood vessel or graft using ultrasound imaging.
5. Loader **2030** for loading device **400** and compressing device anchor **300** into a non-expanded configuration. In one embodiment of the present invention, loader **2030** is a tubular element comprising female luer connectors **2147** and **2150** at both its proximal and distal ends and is used for loading device **400** into delivery sheath **2003** as illustrated at FIG. **20****.** In one embodiment of the present invention, loader **2030** is peel-away. In one embodiment of the present invention, loader **2030** has echogenic orientation markers at the distal end for identification of the long axis of an elliptical cross-section and orientation along the longitudinal axis of the targe blood vessel or graft using ultrasound imaging.
6. In one embodiment of the present invention, delivery element **2080** is a tubular element comprising a female luer connector **2162** at its proximal end and a matching attachment mechanism **2164** at its distal end. Delivery element **2080** can be attached to matching attachment mechanism **327** of device **400** and thus loaded into loader **2030.** In one embodiment of the present invention, delivery element **2080** is a peel-away tubular element such that, after deployment of device **400**, delivery element **2080** is peeled away for removal. In one embodiment of the present invention, delivery element **2080** comprises echogenic orientation markers at the distal end **2164** for identification of the long axis of an elliptical cross-section and orientation along the longitudinal axis of the targe blood vessel or graft using ultrasound imaging.
7. Luer male-to-male connector **2155** for connecting loader **2030** to delivery sheath **2003.**
8. Hemostasis valve **2170** with extension tube **2175** and stopcock **2180** to allow for flushing the delivery system and controlling back-bleeding as illustrated at FIG. **20** and described herein.

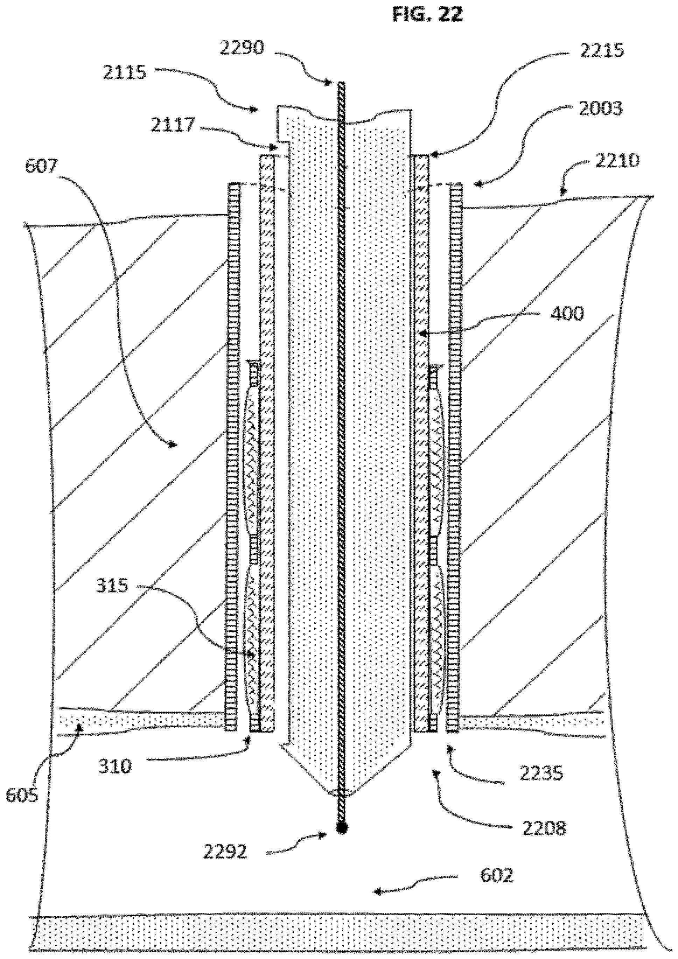

FIG. **22** illustrates an example embodiment of a method for percutaneous and sutureless deployment of device **400** over a guidewire through a procedural sheath according to the present invention. At FIG. **22**, device **400** has been pushed into the delivery sheath **2003** such that the distal tip **310** of the device is flush with the distal tip **2235** of said delivery sheath. In one embodiment of the present invention, device **2215** does not contain a valve.

In order to gain access to the target blood vessel, a guidewire **2290** with an atraumatic tip **2292** has been introduced in the blood vessel **602** through an insertion needle penetrating the tissue **607** and the vessel wall **605.** The insertion needle was retracted to leave distal end **2292** of guidewire **2290** in the blood vessel while the proximal end of guidewire **2290** remained outside the patient's skin **2210.** Dilator / introducer **2115** was introduced in the blood vessel over the guide wire **2290** in order to enlarge hole **2208** in the blood vessel wall **605** and to facilitate the introducing of delivery sheath **2003** containing device **400.**

In one embodiment of the present invention, in order to access blood vessels of small size, e.g., 2 mm in diameter, distal end **2235** of delivery sheath **2003** and the distal end **310** of device **400** need to be placed flush and flush with the inner wall of blood vessel **605.** Thus, the space required for the deployment of the distal anchor segment **315** can be maximized. Flush alignment is verified during the placement procedure using the blood flow back channel **2117** present in the wall of the dilator / introducer **2115.** To flush the distal tip of the device with the blood vessel wall, the ensemble consisting of procedural sheath **2003**, device **400**, and dilator/introducer **2115** is slowly advanced along the guide wire **2290** until control blood flow comes out through blood flow back channel **2117** and is visible at the proximal end of said channel **2117** outside the patient's skin. The correct alignment of the device at the vessel wall can be checked by ultrasound imaging using the echogenic markers of delivery sheath **2003** and/or of device **400.**

In one embodiment of the present invention, in order to maximize blood flow through the device, the deployment of devices of larger sizes than the inner diameter of the targeted blood vessel is achieved by using device having an elliptic cross-section. In such a case, the long axis of the device's elliptical cross-section must be aligned with along the blood vessel, i.e., aligned with the longitudinal axis of the blood vessel. Enhanced ultrasound reflective markers and/or radiopaque markers integrated on the device **400** and/or on delivery sheath **2003** can be used to allow for longitudinal alignment using ultrasound and/or X-ray imaging guidance. Furthermore, a standard dilation of the target blood vessel can be performed if necessary during device deployment to enhance maneuverability of the device.

## Claims

1. A device (400) for attaching an intracorporeal conduit of a hemodialysis system to a blood vessel, the device comprising an intracorporeal conduit and a device anchor configured to suturelessly anastomose the distal end of said intracorporeal conduit to a blood vessel or graft, **characterized in that:**
the intracorporeal conduit (404) provides a fluid path between a hemodialysis machine and a blood vessel or graft and comprises a distal more rigid segment (430) and a proximal less rigid segment (435), wherein said proximal segment is configured to be attached to a vascular access device and said distal segment is configured to be fixedly attached to the tubular body of the device anchor (300) and to extend through the inner lumen thereof such that the distal end of said segment and the distal end of said device anchor are flush; and
the device anchor (300) comprises a tubular body (302) with a pre-shaped self-expandable distal segment (315) configured to self-expand inside said blood vessel or graft or inside a tubular device implanted in the blood vessel or graft, further comprises a pre-shaped self-expandable proximal segment (320), wherein said proximal segment is configured to self-expand outside a wall of said blood vessel or graft, and further comprises a pre-shaped self-expandable waist segment (317) which is about the size of the thickness of the wall of the blood vessel or graft and wherein said distal, proximal, and waist segments are configured to apply sufficient pressure on the wall of the of the blood vessel or graft (510) to keep it steadily attached to said blood vessel or graft wall and are configured to do it without penetrating said wall.

2. The device for attaching an intracorporeal conduit to a blood vessel according to claim **1, characterized in that** the distal segment (430) of said intracorporeal conduit can be made by additional braiding and hardening of the proximal segment of the same intracorporeal conduit (435) or can said distal segment (430) be attached to said proximal segment (435) of said intracorporeal conduit by means of biocompatible heat shrink tubing, overmolding or co-extrusion.

3. The device for attaching an intracorporeal conduit to a blood vessel according to claim **1, characterized in that** the said device anchor (300) is a thin-walled tubular structure made of shape memory material (1610), said distal (1620) and proximal (1615) segments consisting of several longitudinal struts disposed around the circumference of said tubular structure.

4. The device for attaching an intracorporeal conduit to a blood vessel according to claim **3, characterized in that** said distal and the proximal segments have the same or a different number of struts, the struts having the same or different widths and being disposed equidistantly or at variable distance from each other around the circumference of said cylindrical structure (1610).

5. The device for attaching an intracorporeal conduit to a blood vessel according to claim **3, characterized in that** the said device is formed by laser cutting patterns in shape memory material tubing or sheets that are subsequently rolled into cylinders whose edges are welded together.

6. The device for attaching an intracorporeal conduit to a blood vessel according to claim **3, characterized in that** the said longitudinal struts of said distal and proximal segments are pre-shaped through a process of shape setting in their radially expanded and longitudinally compressed configuration (1710).

7. The device for attaching an intracorporeal conduit to a blood vessel according to claim **3, characterized in that** the struts of said proximal and distal segments are coupled to each other by structures extending generally laterally, wherein said coupling structures can be made of shape memory material, woven material or fabric, silicone or polymers, e.g., polyurethanes, PTFE, lightweight polysulphone or of any combination of the above.

8. The device for attaching an intracorporeal conduit to a blood vessel according to claim **6, characterized in that** each longitudinal strut of the proximal segment is bent outwardly as to be set in a V (720) or U-shape (1520).

9. The device for attaching an intracorporeal conduit to a blood vessel according to claim **6, characterized in that** the longitudinal struts of the distal segment (730) of said device have a quasi-rectangular cross-section, have similar (915) or different lengths (925) and are disposed in a circular (910) or an elliptical pattern (920) around a circular or an elliptical inner lumen providing, in the case of an elliptical pattern, for a preferred direction for the alignment of the long axis of the elliptical cross-section of said device anchor along the longitudinal axis of the target blood vessel.

10. The device for attaching an intracorporeal conduit to a blood vessel according to claim **3, characterized in that** the longitudinal struts of the distal segment of said device, when expanded, extend outwardly and perpendicularly to the longitudinal axis of the inner lumen of said device, as to minimize the contact surface between said struts and the inner vessel wall and blood and as to minimize the device weight (1710).

11. The device for attaching an intracorporeal conduit to a blood vessel according to claim **3, characterized in that** the said device anchor is pre-shaped in a strut configuration such that the distal segment (730) of said device anchor has a substantially smaller expanded size than the proximal segment (720) of said device anchor as to minimize the volume of device anchor material inside the blood vessel and to maximize the device fixation capabilities outside the blood vessel.

12. The device for attaching an intracorporeal conduit to a blood vessel according to claim **3, characterized in that** the said device anchor is pre-shaped in a strut configuration such that the proximal segment (720) of said device anchor is pre-shaped at different radial sizes as to accommodate different anatomies and to optimize the device fixation capabilities.

13. The device for attaching an intracorporeal conduit to a blood vessel according to claim **3, characterized in that** the distal segment of said device comprises a plurality of struts forming a net-like structure (1030) that, when pre-shaped, expands radially and compresses longitudinally into the entire cross-section of the target blood vessel or graft (1030), while allowing blood to flow through the net-like structure with minimal turbulence.

14. The device for attaching an intracorporeal conduit to a blood vessel according to claim **3, characterized in that** the said longitudinal struts of the distal (1235) and proximal (1225) segments are of variable width profiles that determine preferred bend locations of the struts, wherein the plane of the waist ring (1230) is at a variable angle with respect to the cross-sectional plane of said cylindrical structure of the device anchor, and wherein the plane of the distal end of said device anchor is parallel to the plane of the waist ring at said variable angle with respect to the cross-sectional plane of said device anchor (1210) and, wherein in one pre-shaped or expanded configuration of said device, the expanded distal and proximal segments are at a variable angle (1430) with respect to the longitudinal axis of the cylindrical structure of said device as to allow that the longitudinal axis of said device anchor to be at a variable angle with respect to the longitudinal axis of the anastomosed blood vessel or graft in expanded configuration (1520).

15. The device for attaching an intracorporeal conduit to a blood vessel according to claim **1, characterized in that** the said intracorporeal conduit comprises, at its distal end, a 3-way valve, infusion, aspiration, and closed (620), that opens under negative or positive pressure.

## Patentansprüche

1. Vorrichtung (400) zum Befestigen einer intrakorporalen Leitung eines Hämodialysesystems an einem Blutgefäß, wobei die Vorrichtung eine intrakorporale Leitung und einen Vorrichtungsanker umfasst so konfiguriert, um das distale Ende der intrakorporalen Leitung zu einem Blutgefäß oder Transplantat nahtlos zu verbinden, **dadurch gekennzeichnet, dass:**
die intrakorporale Leitung (404) einen Flüssigkeitsweg zwischen einer Hämodialysemachine und einem Blutgefäß oder Transplantat bereitstellt und ein distales steiferes Segment (430) und ein proximales weniger starres Segment (435) umfasst, wobei das proximale Segment dazu konfiguriert ist, um an einer Gefäßzugangsvorrichtung befestigt zu werden und das distale Segment dazu konfiguriert ist, um am röhrenförmigen Körper des Vorrichtungsankers (300) fest befestigt zu werden und zu erstrecken durch das innere Lumen des Vorrichtungsankers, so dass das distale Ende des distalen Segments und das distale Ende der Vorrichtungsankers bündig sind;
und
der Vorrichtungsanker (300) einen röhrenförmigen Körper (302) umfasst, mit einem vorgeformten, selbstexpandierenden distalen Segment (315), das dazu konfiguriert ist, sich innerhalb des Blutgefäßes oder Transplantats oder innerhalb eines in das Blutgefäß oder Transplantat implantierten röhrenförmigen Gerätes selbst auszudehnen, ferner ein vorgeformtes, selbstexpandierendes proximales Segment (320) umfasst, das dazu konfiguriert ist, sich außerhalb einer Wand des Blutgefäßes oder des Transplantats selbst auszudehnen, und ferner ein vorgeformtes, selbstexpandierendes Mittelsegment (317) umfasst, das ungefähr die Größe der Wanddicke des Blutgefäßes oder des Transplantats hat und, wobei das distale, das proximale und das Mittelsegment dazu konfiguriert sind, ausreichend Druck auf die Wand des Blutgefäßes oder des Transplantats (510) auszuüben, um die Vorrichtung stabil an der Wand des Blutgefäß oder an der Transplantatwand befestigt zu halten ohne diese Wand zu durchdringen.

2. Vorrichtung zum Befestigen einer intrakorporalen Leitung an einem Blutgefäß nach Anspruch **1, dadurch gekennzeichnet, dass** das distale Segment (430) der intrakorporalen Leitung durch zusätzliches Flechten und Härten des proximalen Segments derselben intrakorporalen Leitung (435) hergestellt werden kann oder dass das distale Segment (430) mittels eines biokompatiblen Schrumpfschlauchs, Überspritzens oder Koextrusion an dem proximalen Segment (435) der intrakorporalen Leitung befestigt werden kann.

3. Vorrichtung zum Befestigen einer intrakorporalen Leitung an einem Blutgefäß nach Anspruch **1, dadurch gekennzeichnet, dass** der Vorrichtungsanker (300) eine dünnwandige röhrenförmige Struktur aus Formgedächtnismaterial (1610) ist, wobei das distale (1620) und das proximale (1615) Segmente aus mehreren Längsstreben bestehen, die um den Umfang der röhrenförmigen Struktur herum angeordnet sind.

4. Vorrichtung zum Befestigen einer intrakorporalen Leitung an einem Blutgefäß nach Anspruch **3**, **dadurch gekennzeichnet, dass** das distale und das proximale Segmente die gleiche oder eine unterschiedliche Anzahl von Streben aufweisen, wobei die Streben die gleichen oder unterschiedlichen Breiten aufweisen und im gleichen Abstand oder in unterschiedlichem Abstand voneinander um den Umfang der zylindrischen Struktur (1610) herum angeordnet sind.

5. Vorrichtung zum Befestigen einer intrakorporalen Leitung an einem Blutgefäß nach Anspruch **3, dadurch gekennzeichnet, dass** die Vorrichtung durch Laserschneiden von Mustern in Formgedächtnismaterial hergestellt wird in Rohren oder Platten, die anschließend zu Zylindern gerollt werden, deren Kanten zusammengeschweißt werden.

6. Vorrichtung zum Befestigen einer intrakorporalen Leitung an einem Blutgefäß nach Anspruch **3**, **dadurch gekennzeichnet, dass** die Längsstreben der distalen und proximalen Segmente durch einen Formgebungsprozess in ihre radial expandierte und longitudinal komprimierte Konfiguration (1710) vorgeformt sind.

7. Vorrichtung zum Befestigen einer intrakorporalen Leitung an einem Blutgefäß nach Anspruch **3**, **dadurch gekennzeichnet, dass** die Streben der proximalen und distalen Segmente durch Strukturen miteinander gekoppelt sind, die sich im Allgemeinen seitlich erstrecken, wobei diese Kopplungsstrukturen aus Formgedächtnismaterial, gewebtem Material oder Gewebe, Silikon oder Polymeren, z. B. Polyurethanen, PTFE, leichtem Polysulfon oder einer beliebigen Kombination der oben genannten Materialien bestehen können.

8. Vorrichtung zum Befestigen einer intrakorporalen Leitung an einem Blutgefäß nach Anspruch **6, dadurch gekennzeichnet, dass** jede Längsstrebe des proximalen Segments nach außen gebogen ist, um eine V- (720) oder U-Form (1520) zu bilden.

9. Vorrichtung zum Befestigen einer intrakorporalen Leitung an einem Blutgefäß nach Anspruch **6, dadurch gekennzeichnet, dass** die Längsstreben des distalen Segments (730) der Vorrichtung einen quasi-rechteckigen Querschnitt aufweisen, ähnliche (915) oder unterschiedliche Längen (925) haben und in einem kreisförmigen (910) oder elliptischen Muster (920) um ein kreisförmiges oder elliptisches Innenlumen angeordnet sind, wobei im Fall eines elliptischen Musters eine bevorzugte Richtung für die Ausrichtung der langen Achse des elliptischen Querschnitts des Vorrichtungsankers entlang der Längsachse des Zielblutgefäßes vorgesehen ist.

10. Vorrichtung zum Befestigen einer intrakorporalen Leitung an einem Blutgefäß nach Anspruch **3**, **dadurch gekennzeichnet, dass** sich die Längsstreben des distalen Segments der Vorrichtung im expandierten Zustand nach außen und senkrecht zur Längsachse des inneren Lumens der Vorrichtung erstrecken, um die Kontaktfläche zwischen den Streben und der inneren Gefäßwand und dem Blut zu minimieren und das Gewicht der Vorrichtung (1710) zu minimieren.

11. Vorrichtung zum Befestigen einer intrakorporalen Leitung an einem Blutgefäß nach Anspruch **3**, **dadurch gekennzeichnet, dass** der Vorrichtungsanker in einer Strebenkonfiguration vorgeformt ist, so dass das distale Segment (730) des Vorrichtungsankers eine wesentlich kleinere expandierte Größe aufweist als das proximale Segment (720) des Vorrichtungsankers, um das Volumen des Vorrichtungsankermaterials im Blutgefäß zu minimieren und die Vorrichtungsfixierungsfähigkeiten außerhalb des Blutgefäßes zu maximieren.

12. Vorrichtung zum Befestigen einer intrakorporalen Leitung an einem Blutgefäß nach Anspruch **3**, **dadurch gekennzeichnet, dass** der Vorrichtungsanker in einer Strebenkonfiguration vorgeformt ist, so dass das proximale Segment (720) des Vorrichtungsankers in verschiedenen radialen Größen vorgeformt ist, um sich an unterschiedliche Anatomien anzupassen und die Vorrichtungsfixierungsfähigkeiten zu optimieren.

13. Vorrichtung zum Befestigen einer intrakorporalen Leitung an einem Blutgefäß nach Anspruch **3, dadurch gekennzeichnet, dass** das distale Segment der Vorrichtung eine Vielzahl von Streben umfasst, die eine netzartige Struktur (1030) bilden, die sich in vorgeformter Form radial ausdehnt und in Längsrichtung auf den gesamten Querschnitt des Zielblutgefäßes oder Transplantats (1030) zusammendrückt, während sie dem Blut ermöglicht, mit minimaler Turbulenz durch die netzartige Struktur zu fließen.

14. Vorrichtung zum Befestigen einer intrakorporalen Leitung an einem Blutgefäß nach Anspruch **3**, **dadurch gekennzeichnet, dass** die Längsstreben des distalen (1235) und des proximalen (1225) Segmente Profile mit variabler Breite aufweisen, die bevorzugte Biegestellen der Streben bestimmen, wobei die Ebene des Mittelrings (1230) in einem variablen Winkel in Bezug auf die Querschnittsebene der zylindrischen Struktur des Vorrichtungsankers steht, und wobei die Ebene des distalen Endes des Vorrichtungsankers parallel zur Ebene des Mittelrings in dem variablen Winkel in Bezug auf die Querschnittsebene des Vorrichtungsankers (1210) steht, und wobei in einer vorgeformten oder erweiterten Konfiguration der Vorrichtung die erweiterten distalen und proximalen Segmente in einem variablen Winkel (1430) in Bezug auf die Längsachse der zylindrischen Struktur der Vorrichtung stehen, um zu ermöglichen, dass die Längsachse des Vorrichtungsankers in erweiterter Konfiguration (1520) in einem variablen Winkel in Bezug auf die Längsachse des anastomosierten Blutgefäßes oder Transplantats steht.

15. Vorrichtung zum Befestigen einer intrakorporalen Leitung an einem Blutgefäß nach Anspruch **1**, **dadurch gekennzeichnet, dass** die intrakorporale Leitung an ihrem distalen Ende ein 3-Wege- Ventil (Infusion, Aspiration und geschlossen) (620) aufweist, das sich bei negativem oder positivem Druck öffnet.

## Revendications

1. Dispositif (400) de fixation d'un conduit intracorporel d'un système d'hémodialyse à un vaisseau sanguin, ledit dispositif comprenant un conduit intracorporel et une ancre du dispositif configurée pour anastomoser sans suture l'extrémité distale dudit conduit intracorporel à un vaisseau sanguin ou à une prothèse vasculaire, **caractérisé en ce que** :
le conduit intracorporel (404) fournit un trajet de fluide entre une machine d'hémodialyse et un vaisseau sanguin ou une prothèse vasculaire et comprend un segment distal plus rigide (430) et un segment proximal moins rigide (435), dans lequel ledit segment proximal est configuré pour être connecté à un dispositif d'accès vasculaire et ledit segment distal est configuré pour être attaché de manière fixe au corps tubulaire de l'ancre du dispositif (300) et pour s'étendre à travers la lumière interne de ladite ancre du dispositif de sorte que l'extrémité distale dudit segment et l'extrémité distale de ladite ancre du dispositif affleurent ; et
l'ancre du dispositif (300) comprend un corps tubulaire (302) avec un segment distal auto-expansible préformé (315) configuré pour s'étendre automatiquement à l'intérieur dudit vaisseau sanguin ou de ladite prothèse vasculaire ou à l'intérieur d'un dispositif tubulaire implanté dans le vaisseau sanguin ou dans la prothèse vasculaire, comprend en outre un segment proximal auto-expansible préformé (320), dans lequel ledit segment proximal est configuré pour s'étendre automatiquement à l'extérieur d'une paroi dudit vaisseau sanguin ou de ladite prothèse vasculaire, et comprend en outre un segment médian auto-expansible préformé (317) qui a approximativement la même dimension que l'épaisseur de la paroi du vaisseau sanguin ou de la prothèse vasculaire et dans lequel lesdits segments distal, proximal et médian sont configurés pour appliquer une pression suffisante sur la paroi du vaisseau sanguin ou de la prothèse vasculaire (510) afin de maintenir ledit dispositif stablement fixé à ladite paroi du vaisseau sanguin ou de la prothèse vasculaire et sont configurés pour ce faire sans pénétrer dans ladite paroi.

2. Dispositif de fixation d'un conduit intracorporel à un vaisseau sanguin selon la revendication **1, caractérisé en ce que** le segment distal (430) dudit conduit intracorporel peut être réalisé par le tressage et le durcissement supplémentaires du segment proximal du même conduit intracorporel (435) ou ledit segment distal (430) peut être fixé audit segment proximal (435) dudit conduit intracorporel au moyen d'une gaine thermorétractable biocompatible, d'un surmoulage ou d'une coextrusion.

3. Dispositif de fixation d'un conduit intracorporel à un vaisseau sanguin selon la revendication **1, caractérisé en ce que** ladite ancre du dispositif (300) est une structure tubulaire à paroi mince constituée d'un matériau à mémoire de forme (1610), lesdits segments distal (1620) et proximal (1615) de ladite ancre étants constitués de plusieurs entretoises longitudinales disposées autour de la circonférence de ladite structure tubulaire.

4. Dispositif de fixation d'un conduit intracorporel à un vaisseau sanguin selon la revendication **3**, **caractérisé en ce que** lesdits segments distal et proximal ont le même nombre ou un nombre différent d'entretoises, les entretoises ayant des largeurs identiques ou différentes et étant disposées à égale distance ou à distance variable les unes des autres autour de la circonférence de ladite structure cylindrique (1610).

5. Dispositif de fixation d'un conduit intracorporel à un vaisseau sanguin selon la revendication **3**, **caractérisé en ce que** ledit dispositif est formé par des motifs découpés au laser dans des tubes ou des feuilles en un matériau à mémoire de forme qui sont ensuite roulés en cylindres dont les bords sont soudés ensemble.

6. Dispositif de fixation d'un conduit intracorporel à un vaisseau sanguin selon la revendication **3**, **caractérisé en ce que** lesdites entretoises longitudinales desdits segments distal et proximal sont préformées par un processus de mise en forme dans leur configuration radialement étendue et longitudinalement comprimée (1710).

7. Dispositif de fixation d'un conduit intracorporel à un vaisseau sanguin selon la revendication **3**, **caractérisé en ce que** les entretoises desdits segments proximal et distal sont couplées l'une à l'autre par des structures s'étendant généralement latéralement, dans lequel lesdites structures de couplage peuvent être constituées d'un matériau à mémoire de forme, d'un matériau ou d'un tissu tissé, du silicone ou des polymères, par exemple des polyuréthanes, du PTFE, d'un polysulfone léger ou de toute combinaison de ceux-ci.

8. Dispositif de fixation d'un conduit intracorporel à un vaisseau sanguin selon la revendication **6, caractérisé en ce que** chaque entretoise longitudinale du segment proximal est pliée vers l'extérieur de manière à être mise en forme de V (720) ou de U (1520).

9. Dispositif de fixation d'un conduit intracorporel à un vaisseau sanguin selon la revendication **6, caractérisé en ce que** les entretoises longitudinales du segment distal (730) dudit dispositif ont une section quasi rectangulaire, ont des longueurs similaires (915) ou différentes (925) et sont placées selon un motif circulaire (910) ou elliptique (920) autour d'une lumière interne circulaire ou elliptique fournissant, dans le cas d'un motif elliptique, une direction privilégiée pour l'alignement du grand axe de la section transversale elliptique de ladite ancre du dispositif le long de l'axe longitudinal du vaisseau sanguin cible.

10. Dispositif de fixation d'un conduit intracorporel à un vaisseau sanguin selon la revendication **3, caractérisé en ce que** les entretoises longitudinales du segment distal dudit dispositif, lorsqu'elles sont étendues, s'étendent vers l'extérieur et perpendiculairement à l'axe longitudinal de la lumière interne dudit dispositif, de manière à minimiser la surface de contact entre lesdites entretoises et la paroi interne du vaisseau et le sang et minimiser le poids du dispositif (1710).

11. Dispositif de fixation d'un conduit intracorporel à un vaisseau sanguin selon la revendication **3**, **caractérisé en ce que** ladite ancre du dispositif est préformée dans une configuration d'entretoise de sorte que le segment distal (730) de ladite ancre du dispositif a une taille étendue sensiblement plus petite que le segment proximal (720) de ladite ancre du dispositif de manière à minimiser le volume du matériau de l'ancre du dispositif à l'intérieur du vaisseau sanguin et maximiser les capacités de fixation du dispositif à l'extérieur du vaisseau sanguin.

12. Dispositif de fixation d'un conduit intracorporel à un vaisseau sanguin selon la revendication **3, caractérisé en ce que** ladite ancre du dispositif est préformée dans une configuration d'entretoise de sorte que le segment proximal (720) de ladite ancre du dispositif est préformé à des tailles radiales différentes de manière à s'adapter à des anatomies différentes et optimiser les capacités de fixation du dispositif.

13. Dispositif de fixation d'un conduit intracorporel à un vaisseau sanguin selon la revendication **3, caractérisé en ce que** le segment distal dudit dispositif comprend une pluralité d'entretoises formant une structure en forme de filet (1030) qui, une fois préformée, s'étend radialement et se comprime longitudinalement dans toute la section transversale du vaisseau sanguin ou de la prothèse vasculaire cible (1030), tout en permettant au sang de s'écouler à travers la structure en forme de filet avec une turbulence minimale.

14. Dispositif de fixation d'un conduit intracorporel à un vaisseau sanguin selon la revendication **3**, **caractérisé en ce que** les dites entretoises longitudinales des segments distal (1235) et proximal (1225) ont des profils de largeur variable qui déterminent les emplacements préfères de pliage des entretoises, dans lequel le plan de l'anneau médian (1230) forme un angle variable par rapport au plan de coupe transversale de ladite structure cylindrique de l'ancre du dispositif, et dans lequel le plan de l'extrémité distale de ladite ancre du dispositif est parallèle au plan de l'anneau médian audit angle variable par rapport au plan de coupe transversale de ladite ancre du dispositif (1210) et, dans lequel dans une configuration préformée ou étendue dudit dispositif, les segments distal et proximal étendus forment un angle variable (1430) par rapport à l'axe longitudinal de la structure cylindrique dudit dispositif de manière à permettre à l'axe longitudinal de ladite ancre du dispositif de former un angle variable par rapport à l'axe longitudinal du vaisseau sanguin ou de la prothèse vasculaire anastomosés en configuration étendue (1520).

15. Dispositif de fixation d'un conduit intracorporel à un vaisseau sanguin selon la revendication **1**, **caractérisé en ce que** ledit conduit intracorporel comprend, à son extrémité distale, une valve à 3 voies, perfusion, aspiration et fermée (620), qui s'ouvre sous pression négative ou sous pression positive.
